# EUROPEAN PATENT APPLICATION

(11) **EP 1 621 535 A1**
(43) Date of publication of application: **01.02.2006**
(21) Application number: 04291903.5
(22) Date of filing: 27.07.2004
(51) Int. Cl.: C07D 401/06, C07D 409/14, A61K 31/4178, A61K 31/513, A61K 31/551

(54) **Substituted cyclic urea derivatives, preparation thereof and pharmaceutical use thereof as kinase inhibitors**

(71) Applicant: Aventis Pharma S.A., 92160 Antony (FR)
(72) Inventor: Strobel, Hartmut, 65835 Liederbach (DE); Nemecek, Conception, 94320 Thiais (FR); Lesuisse, Dominique, 93100 Montreuil (FR); Ruf, Sven, 65439 Flôrsheim (DE); El-Ahmad Youssef, 94000 Creteil (FR); Mauger, Jacques, 85704 AZ Tucson (US); Guessregen, Stefan, 65205 Wiesbaden (DE); Ritter, Kurt, 60594 Frankfurt am Main (DE); Malleron, Jean-Luc, 91460 Marcoussis (FR)
(74) Representative: Rousseau, Pierrick Edouard

(57) **Abstract**

The invention relates to the products of formula (I):

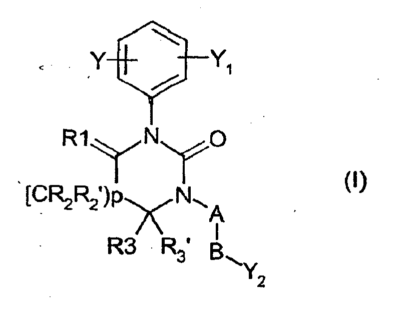

## Description

The present invention relates to novel substituted cyclic urea derivatives, to a process for preparing them, to their use as medicinal products, to pharmaceutical compositions containing them and to the pharmaceutical use of such derivatives for preventing and treating complaints that may be modulated by inhibiting the activity of protein kinases.

The present invention relates to novel substituted cyclic urea derivatives that are substituted with heterocycles and have inhibitory effects on protein kinases.

The products of the present invention may thus be used especially for preventing or treating complaints capable of being modulated by inhibiting the activity of protein kinases.

The inhibition and regulation of protein kinases especially constitute a powerful new mechanism of action for treating a large number of solid tumours.

Such complaints that the products of the present patent application can treat are thus most particularly solid tumours.

Such protein kinases belong especially to the following group:
IGF1, Raf, EGF, PDGF, VEGF, Tie2, KDR, Flt1-3, FAK, Src, Abl, cKit, cdk1-9, Auroral-2, cdc7, Akt, Pdk, S6K, Jnk, IR, FLK-1, FGFR1, FGFR2, FGFR3, FGFR4, FGFR5, PLK, Pyk2, CDK7, CDK2 et EGFR.

Such protein kinases belong more especially to the following group: IGF1, cdc7, Auroral-2, Src, Jnk, FAK, KDR, IR, Tie2, CDK7, CDK2 et EGFR.
The protein kinase IGF1-R (Insulin Growth Factor-1 Receptor) is particularly indicated.

The protein kinase FAK is also indicated.

The protein kinase AKT is also indicated.

The present invention thus relates particularly to novel inhibitors of the IGF-1R receptor that may be used for oncology treatments.

The present invention also relates to novel FAK receptor inhibitors that may be used for oncology treatments.

The present invention also relates to novel AKT receptor inhibitors that may be used for oncology treatments.

Cancer remains a disease for which the existing treatments are clearly insufficient. Certain protein kinases, especially including IGF-1R (Insulin Growth Factor 1 Receptor), play an important role in many cancers. The inhibition of such protein kinases is potentially important in the chemotherapy of cancers, especially for suppressing the growth or survival of tumours. The present invention thus relates to the identification of novel products that inhibit such protein kinases.

Protein kinases participate in signalling events that control the activation, growth and differentiation of cells in response either to extracellular mediators or to changes in the environment. In general, these kinases belong to two groups: those that preferentially phosphorylate serine and/or threonine residues and those that preferentially phosphorylate tyrosine residues [S.K. Hanks and T. Hunter, FASEB. J., 1995, 9, pages 576-596]. The serine/threonine kinases are, for example, the isoforms of the protein kinases C [A.C. Newton, J. Biol. Chem., 1995, 270, pages 28495-28498] and a group of cycline-dependent kinases, for instance cdc2 [J. Pines, Trends in Biochemical Sciences, 1995, 18, pages 195-197]. Tyrosine kinases comprise growth factor receptors, for instance the epidermal growth factor (EGF) receptor [S. Iwashita and M. Kobayashi, Cellular Signalling, 1992, 4, pages 123-132], and cytosol kinases, for instance p56tck, p59fYn and ZAP-70 and the kinases csk [C. Chan et. al., Ann. Rev. Immunol., 1994, 12, pages 555-592].

Abnormally high levels of kinase protein activity have been implicated in many diseases, resulting from abnormal cellular functions. This may arise either directly or indirectly from a dysfunction in the mechanisms for controlling the kinase activity, linked, for example, to a mutation, an overexpression or an inappropriate activation of the enzyme, or an over- or underproduction of cytokines or of growth factors, also involved in the transduction of the signals upstream or downstream of the kinases. In all these cases, a selective inhibition of the action of the kinases offers hope of a beneficial effect.

The type 1 receptor for the insulin-like growth factor (IGF-I-R) is a transmembrane receptor with tyrosine kinase activity, which binds firstly to IGFI, but also to IGFII and to insulin with lower affinity. The binding of IGF1 to its receptor results in oligomerization of the receptor, the activation of tyrosine kinase, intermolecular autophosphorylation and the phosphorylation of cell substrates (main substrates: IRS1 and Shc). The receptor activated by its ligand induces mitogenic activity in normal cells. However, IGF-I-R plays an important role in "abnormal" growth.

Several clinical reports underline the important role of the IGF-I route in the development of human cancers:

IGF-I-R is often found overexpressed in many types of tumour (breast, colon, lung, sarcoma, etc.) and its presence is often associated with a more aggressive phenotype.

High concentrations of circulating IGF1 are strongly correlated with a risk of prostate cancer, lung cancer and breast cancer.

Furthermore, it has been widely documented that IGF-I-R is necessary for establishing and maintaining the transformed phenotype in vitro as in vivo [Baserga R, Exp. Cell. Res., 1999, 253, pages 1-6]. The kinase activity of IGF-I-R is essential for the transformation activity of several oncogenes: EGFR, PDGFR, the large T antigen of the SV40 virus, activated Ras, Raf, and v-Src. The expression of IGF-I-R in normal fibroblasts induces a neoplastic phenotype, which may then result in the formation of a tumour in vivo. The expression of IGF-I-R plays an important role in substrate-independent growth. IGF-I-R has also been shown to be a protector in chemotherapy-induced and radiation-induced apoptosis, and cytokine-induced apoptosis. Furthermore, the inhibition of endogenous IGF-I-R with a negative dominant, the formation of a triple helix or the expression of an antisense sequence brings about suppression of the transforming activity in vitro and reduction of tumour growth in animal models.

Among the kinases for which a modulation of the activity is desired, FAK (Focal Adhesion Kinase) is also a preferred kinase.

FAK is a cytoplasmic tyrosine kinase that plays an important role in transducing the signal transmitted by the integrins, a family of heterodimeric receptors of cellular adhesion. FAK and the integrins are colocalized in perimembrane structures known as adhesion plaques. It has been shown in many cell types that the activation of FAK and its phosphorylation on tyrosine residues and in particular its autophosphorylation on tyrosine 397 were dependent on the binding of the integrins to their extracellular ligands and thus induced during cellular adhesion [Kornberg L, et al. J. Biol. Chem. 267(33): 23439-442 (1992)]. The autophosphorylation on tyrosine 397 of FAK represents a binding site for another tyrosine kinase, Src, via its SH2 domain [Schaller et al. Mol. Cell. Biol. 14: 1680-1688 1994; Xing et al. Mol. Cell. Biol. 5: 413-421 1994]. Src can then phosphorylate FAK on tyrosine 925, thus recruiting the adapter protein Grb2 and inducing in certain cells activation of the ras and MAP kinase pathway involved in controlling cellular proliferation [Schlaepfer et al. Nature; 372: 786-791 1994; Schlaepfer et al. Prog. Biophy. Mol. Biol. 71: 435-478 1999; Schlaepfer and Hunter, J. Biol. Chem. 272: 13189-13195 1997].

The activation of FAK can thus induce the jun NH2-terminal kinase (JNK) signalling pathway and result in the progression of the cells to the G1 phase of the cellular cycle [Oktay et al., J. Cell. Biol. 145: 1461-1469 1999]. Phosphatidylinositol-3-OH kinase (PI3-kinase) also binds to FAK on tyrosine 397 and this interaction might be necessary for the activation of PI3-kinase [Chen and Guan, Proc. Nat. Acad. Sci. USA. 91: 10148-10152 1994; Ling et al. J. Cell. Biochem. 73: 533-544 1999]. The FAK/Src complex phosphorylates various substrates, for instance paxillin and p130CAS in fibroblasts [Vuori et al. Mol. Cell. Biol. 16: 2606-2613 1996].

The results of numerous studies support the hypothesis that FAK inhibitors might be useful in treating cancer. Studies have suggested that FAK might play an important role in *in vitro* cell proliferation and/or survival. For example, in CHO cells, certain authors have demonstrated that the overexpression of p125FAK induces an acceleration of the G1 to S transition, suggesting that p125FAK promotes cellular proliferation [Zhao J.-H et al. J. Cell Biol. 143: 1997-2008 1998]. Other authors have shown that tumour cells treated with FAK antisense oligonucleotides lose their adhesion and go into apoptosis (Xu and al, Cell Growth Differ. 4: 413-418 1996). It has also been demonstrated that FAK promotes the migration of cells *in vitro*. Thus, fibroblasts that are deficient for the expression of FAK ("knockout" mice for FAK) show a rounded morphology and deficiencies in cell migration in response to chemotactic signals, and these defects are suppressed by reexpression of FAK [DJ. Sieg et al., J. Cell Science. 112: 2677-91 1999]. The overexpression of the C-terminal domain of FAK (FRNK) blocks the stretching of adherent cells and reduces cellular migration *in vitro* [Richardson A. and Parsons J.T. Nature. 380: 538-540 1996]. The overexpression of FAK in CHO or COS cells or in human astrocytoma cells promotes migration of the cells. The involvement of FAK in promoting the proliferation and migration of cells in numerous cell types in vitro suggests the potential role of FAK in neoplastic processes. A recent study has effectively demonstrated the increase in the proliferation of tumour cells in vivo after induction of the expression of FAK in human astrocytoma cells [Cary L.A. et al. J. Cell Sci. 109: 1787-94 1996; Wang D et al. J. Cell Sci. 113: 4221-4230 2000]. Furthermore, immunohistochemical studies on human biopsies have demonstrated that FAK is overexpressed in prostate cancer, breast cancer, thyroid cancer, cancer of the colon, melanoma, brain cancer and lung cancer, the level of expression of FAK being directly correlated to the tumours having the most aggressive phenotype [Weiner TM, et al. Lancet. 342 (8878): 1024-1025 1993 ; Owens et al. Cancer Research. 55: 2752-2755 1995; Maung K. et al. Oncogene 18: 6824-6828 1999; Wang D et al. J. Cell Sci. 113: 4221-4230 2000].

Protein kinase AKT (also known as PKB) and phosphoinositide 3-kinase (PI3K) are involved in a cell signalling pathway that transmits signals from growth factors activating membrane receptors.

This transduction pathway is involved in numerous cellular functions: regulation of apoptosis, control of transcription and translation, glucose metabolism, angiogenesis and mitochondrial integrity. First identified as an important component of insulin-dependent signalling pathways regulating metabolic responses, serine/threonine kinase AKT was then identified as a mediator playing a key role in survival induced with growth factors. It has been shown that AKT can inhibit death by apoptosis induced by various stimuli, in a certain number of cell types and tumour cells. In accordance with these findings, it has been shown that AKT can, by phosphorylation of given serine residues, inactivate BAD, GSK3□, caspase-9, and Forkhead transcription factor, and can activate IKKalpha and e-NOS. It is interesting to note that the protein BAD is found hyper-phosphorylated in 11 human tumour cell lines out of 41 studied. Furthermore, it has been shown that hypoxia modulates the induction of VEGF in cells transformed with Ha-ras by activating the PI3K/AKT pathway and by involving the binding sequence of the HIF-1 (hypoxia inducible factor-1) transcription factor known as HRE for "hypoxy-responsive element".

AKT plays a very important role in cancer pathologies. The amplification and/or overexpression of AKT has been reported in many human tumours, for instance gastric carcinoma (amplification of AKT1), ovary carcinoma, breast carcinoma or pancreatic carcinoma (amplification and overexpression of AKT2) and breast carcinomas deficient in oestrogen receptors, and also androgen-independent prostate carcinomas (overexpression of AKT3). Furthermore, AKT is constitutively activated in all the PTEN (-/-) tumours, the PTEN phosphatase being deleted or inactivated by mutations in many types of tumours, for instance carcinomas of the ovary, of the prostate, of the endometrium, glioblastomas and melanomas. AKT is also involved in the oncogenic activation of bcr-abl (references: Khawaja A., Nature 1999, 401, 33-34; Cardone et al. Nature 1998, 282, 1318-1321; Kitada S. et al., Am. J. Pathol. 1998 Jan; 152(1): 51-61 ; Mazure NM et al. Blood 1997, 90, 3322-3331; Zhong H. et al. Cancer Res. 2000, 60, 1541-1545).

One subject of the present invention is thus the products of general formula (I): 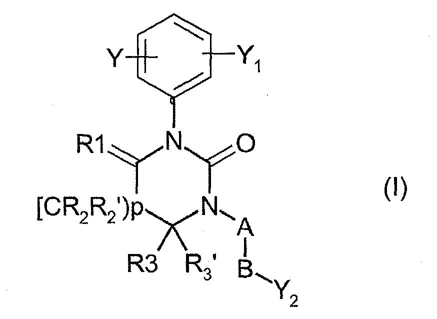
in which
R1 represents O or NH,
p represents an integer from 0 to 2,
Y and Y1, which may be identical or different, are such that one from among Y and Y1 is chosen from:
alkyl containing 1 to 7 carbon atoms optionally substituted with one or more radicals, which may be identical or different, chosen from halogen atoms, cycloalkyl and the hydroxyl radical;
3- to 7-membered cycloalkyl optionally substituted with one or more radicals, which may be identical or different, chosen from fluorine atoms and alkyl radicals containing 1 to 3 carbon atoms;
alkylamino, optionally substituted with one or more halogen atoms;
dialkylamino, optionally substituted with one or more radicals, which may be identical or different, chosen from halogen atoms and alkoxy radicals and in which the two alkyl residues may optionally form, together with the nitrogen atom to which they are attached, a 4- to 10-membered heterocycle optionally containing one or more other hetero atoms, which may be identical or different, chosen from O, N, NR4 and S and optionally substituted with one or more radicals, which may be identical or different, chosen from halogen atoms and alkyl and alkoxy radicals; phenylalkylamino optionally substituted on alkyl or phenyl; arylmercapto or heteroarylmercapto, optionally substituted with one or more radicals, which may be identical or different, chosen from halogen atoms and alkyl and alkoxy radicals;
and the other from among Y and Y1 is chosen from these same values and in addition from the following values: hydrogen; halogen; hydroxyl; nitro; CN; SF5; NR5R6; optionally substituted alkyl, alkenyl and alkoxy; optionally substituted aryl and heteroaryl; O-alkenyl ; O-alkynyl ; O-cycloalkyl ; S(O)n-alkyl ; S(O)n-alkenyl ; S(O) n-alkynyl ; S (O) n-cycloalkyl ; S (O) nNR5R6 ; free, salified or esterified carboxyl and CONR5R6 ;
p represents the integers 0, 1 and 2,
R2, R2' , R3 and R3', which may be identical or different, represent hydrogen, halogen, alkyl, alkenyl, alkynyl, cycloalkyl, cycloalkylalkyl, aryl and heteroaryl which are optionally substituted, or alternatively two of the residues R2, R2', R3 and R3' form, together with the carbon atom(s) to which they are attached, a carbocyclic or heterocyclic radical, these radicals being 3- to 10-membered and the heterocyclic radical containing one or more hetero atoms chosen from O, S, N and NR4, all these radicals optionally being substituted,
A represents a single bond; an alkylene radical; an alkenyl radical; alkynyl; CO; SO2; O; NH; NH-alkyl;
B represents a saturated or unsaturated monocyclic or bicyclic heterocyclic radical containing one or more hetero atoms, which may be identical or different, chosen from O, S, N and NR4, optionally substituted with one or more substituents, which may be identical or different, chosen from the values of Y2;
Y2 represents hydrogen; halogen; hydroxyl; cyano; alkyl; alkoxy ;cycloalkyl ; heterocycloalkyl ; aryl ; heteroaryl ; -O-alkenyl; -O-alkynyl ; -O-cycloalkyl ; -S(O)n-alkyl ; -S(O)n-alkenyl ; -S(O)n-alkynyl ; S(O)n-cycloalkyl ; COOR13 ; -OCOR13 ; NR5R6 ; CONR5R6 ; S(O)n-NRSR6 ; -NR10-CO-R13 ; -NR10-SO2-R13 ; NH-SO2-NR5R6 ; -NR10-CO-NR5R6 ; -NR10-CS-NR5R6 and -NR10-COOR13 ; all these radicals being optionally substituted;
R4 represents a hydrogen atom or an alkyl, alkenyl, cycloalkyl, alkylCO, alkylSO₂ or aryle, all optionally substituted;
R5 and R6, which may be identical or different, are chosen from hydrogen; alkyl, alkenyl, alkynyl, cycloalkyl, cycloalkenyl, heterocycloalkyl, optionally substituted aryl and heteroaryl; or alternatively R5 and R6 form, with the nitrogen atom to which they are attached, a 3- to 10-membered heterocyclic radical containing one or more hetero atoms chosen from O, S, N and optionally substituted NR4;
all the above alkyl, alkenyl, alkynyl and alkoxy radicals being linear or branched and containing up to 6 carbon atoms;
all the above cycloalkyl and heterocycloalkyl radicals containing up to 7 carbon atoms,
all the above aryl and heteroaryl radicals containing up to 10 carbon atoms;
all the above alkyl, alkenyl, alkynyl and alkoxy radicals cycloalkyl, heterocycloalkyl, aryl and heteroaryl radicals, carbocyclic and heterocyclic radicals, and also the ring formed by R5 and R6 with the atom to which they are attached being optionally substituted with one or more radicals, which may be identical or different, chosen from halogen atoms; cyano; hydroxyl; alkoxy; CF3 ; nitro; aryl; heteroaryl; -C(=O)-OR9; -C(=O)-R8; -NR11R12; -C (=O) -NR11R12 ; -N (R10) -C (=O) -R8 ; -N (R10) -C (=O) -OR9 ; N(R10) -C (=O) -NR11R12 ; -N (R10) -S (O) n-R8 ; -S (O) n-R8; -N(R10)-S(O)n-NR11R12 and -S(O)n-NR11R12 radicals;
all the above aryl and heteroaryl radicals also being optionally substituted with one or more radicals chosen from alkyl, phenylalkyl, alkoxy and alkylenedioxy radicals;
all the above cyclic radicals and also the ring formed by R5 and R6 with the atom to which they are attached being also optionally substituted with one or more radicals chosen from oxo and thioxo;
n represents an integer from 0 to 2,
R8 represents alkyl, alkenyl, cycloalkyl, cycloalkylalkyl, heterocycloalkyl, heterocycloalkyl-alkyl, aryl, arylalkyl, heteroaryl and heteroarylalkyl ; all these radicals being optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
R9 represents the values of R8 and hydrogen;
R10 represents hydrogen or alkyl;
R11 and R12, which may be identical or different, represent hydrogen; alkyl, cycloalkyl and phenyl optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
or alternatively R11 and R12 form, with the nitrogen atom to which they are attached, a 5- to 7-membered cyclic radical containing one or more hetero atoms chosen from O, S, N and NR14 and preferably a cyclic amine, optionally substituted with one or more radicals, which may be identical or different, chosen from halogen atoms and cyano, hydroxyl, alkoxy, alkyl, CF3, nitro, phenyl, phenylalkyl and free, salified, esterified or amidated carboxyl radicals;
R13, which may be identical to or different from R5 or R6, being chosen from the values of R5 or R6;
the said products of formula (I) being in all the possible racemic, enantiomeric and diastereoisomeric isomer forms, and also the addition salts with mineral and organic acids or with mineral and organic bases of the said products of formula (I).

A subject of the present invention is, more specifically, the products of formula (I) as defined above corresponding to formula (Ia): 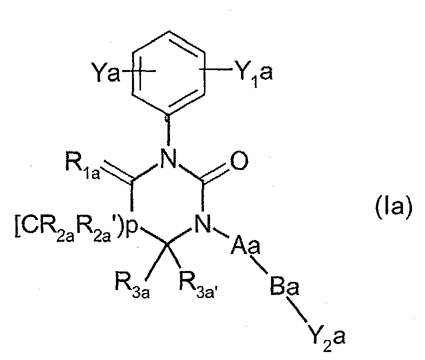
in which:
R1a stands for O or NH,
p represents an integer from 0 to 2,
Ya and Y1a, which may be identical or different, are such that one from among Ya and Y1a is chosen from:
   alkyl containing 1 to 7 carbon atoms optionally substituted with one or more radicals, which may be identical or different, chosen from fluorine atoms and the radical hydroxyl ; 3- to 7-membered cycloalkyl optionally substituted with one or more radicals, which may be identical or different, chosen from fluorine atoms,alkyl radicals containing 1 to 3 carbon atoms, cyclopropyl;
   alkylamino, optionally substituted with one or more fluorine atoms; dialkylamino, optionally substituted with one or more radicals, which may be identical or different, chosen from fluorine atoms and alkoxy radicals and in which the two alkyl residues may optionally form, together with the nitrogen atom to which they are attached, a 4- to 10-membered heterocycle optionally containing one or more other hetero atoms, which may be identical or different, chosen from O, N, Nalkyl and S and optionally substituted with one or more radicals, which may be identical or different, chosen from fluorine atoms and alkyl and alkoxy radicals; phenylalkylamino optionally substituted on alkyl or phenyl; 5- to 6-membered phenylmercapto or heteroarylmercapto, optionally substituted with one or more radicals, which may be identical or different, chosen from halogen atoms and alkyl and alkoxy radicals;
   and the other from among Ya and Y1a is chosen from these same values and in addition from the following values: hydrogen; halogen; hydroxyl ; nitro; CN; SF5 ; NR5aR6a; optionally substituted alkyl, alkenyl and alkoxy; O-allyl; O-propynyl ; O-cycloalkyl ; CF3 ; optionally substituted phenyl and heteroaryl; -S(O)n-alkyl, S(O)n-allyl; S(O)n-propynyl ; SO2NR5aR6a ; S(O)n-cycloalkyl ; free, salified or esterified carboxyl; CONR5aR6a ;
   R2a, R2a', R3a, R3a' represent hydrogen, halogen , and alkyl, it being understood that two of the substituents R2a, R2a', R3a, R3a' can form, together with the carbon atom(s) to which they are attached, a 3- to 6-membered cycloalkyl or heterocycloalkyl radical containing a nitrogen atom, all these radicals being optionally substituted,
   Aa represents a single bond; an alkylene radical; CO; SO2; O; NH; NH-alkyl;
   Ba represents pyridyl, pyrimidinyl, quinolyl, azaindolyl, quinazolyl, thiazolyl, imidazolyl, pyrazolyl, furazanyl, isoxazolyl, morpholinyl, pyrrolidinyl, furyl, piperidyl, thienyl, chromenyl, oxochromenyl, indolyl, pyrrolyl, purinyl, benzoxazinyl, benzimidazolyl, indazolyl and benzofuryl radicals, these radicals being optionally substituted with one or more radicals chosen from the values of Y2a;
   Y2a represents hydrogen; halogen; hydroxyl; alkyl; alkoxy ; cycloalkyl ; heterocycloalkyl; aryl ; heteroaryl ; O-allyl; O-propynyl; O-cycloalkyl; S(O)n-alkyl; S(O)n-allyl; S(O)n-propynyl; S(O)n-cycloalkyl; COOR9a; OCOR8a; -NR10a-CO-NR5aR6a; NR5aR6a; CONR5aR6a; S(O)n-R5aR6a; NHCOR8a; NH-S(O)nR8a; NH-S(O)nCF3; NH-SO2-NR5aR6a, all these radicals being optionally substituted;
   R4a represents a hydrogen atom or an alkyl, cycloalkyl, or phenyl radicals, all optionally substituted with one or more substituents, which may be identical or different, chosen from halogen atoms; hydroxyl; alkoxy; dialkylamino; aryl and heteroaryl radicals, these last two radicals optionally substituted with one or more substituents, which may be identical or different, chosen from halogen atoms and alkyl and alkoxy radicals,
   R5a and R6a, which may be identical or different, are chosen from hydrogen, alkyl, alkenyl, cycloalkyl, cycloalkenyl, heterocycloalkyl, optionally substituted aryl and heteroaryl; or alternatively R5a and R6a form, with the nitrogen atom to which they are attached, a 3-to 10-membered heterocyclic radical containing one or more hetero atoms chosen from O, S, N and optionally substituted NR4a;
   all the above alkyl, alkenyl, alkynyl and alkoxy radicals being linear or branched and containing up to 6 carbon atoms;
   all the above cycloalkyl and heterocycloalkyl radicals containing up to 7 carbon atoms,
   all the above aryl and heteroaryl radicals containing up to 10 carbon atoms;
   all the above alkyl, alkenyl, alkynyl, alkoxy, cycloalkyl, heterocycloalkyl, aryl, heteroaryl, carbocyclic and heterocyclic radicals being optionally substituted with one or more radicals, which may be identical or different, chosen from halogen atoms; cyano; hydroxyl; alkoxy; CF3; nitro; aryl; heteroaryl; -C(=O)-OR9a; -C(=O)-R8a; -NR11aR12a; -C(=O)-NR11aR12a; -N (R10a) - C (=O) -R8a; -N(R10a)-C(=O)-OR9a; N(R10a)-C(=O)-NR11aR12a; -N(R10a)-S(O)n-R8a; -S(O)n-R8a; -N(R10a)-S(O)n-NR11aR12a and -S(O)n-NR11aR12a radicals;
   all the above aryl and heteroaryl radicals also being optionally substituted with one or more radicals chosen from alkyl, phenylalkyl and alkylenedioxy radicals;
   n represents an integer from 0 to 2,
   R8a represents alkyl, alkenyl, cycloalkyl, cycloalkylalkyl, heterocycloalkyl, heterocycloalkyl-alkyl, phenyl, phenylalkyl, heteroaryl and heteroarylalkyl; all these radicals being optionally substituted with one or more radicals, which may be identical or different, chosen from halogen atoms and hydroxyl, alkoxy, alkyl, CF3, nitro, phenyl and free, salified, esterified or amidated carboxyl radicals;
   R9a represents the values of R8 and hydrogen;
   R10a represents hydrogen or alkyl;
   R11a and R12a, which may be identical or different, represent hydrogen, alkyl, cycloalkyl, cycloalkylalkyl, phenyl and phenylalkyl optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
   or alternatively R11a and R12a form, with the nitrogen atom to which they are attached, a cyclic radical chosen from pyrrolidinyl, piperidyl, piperazinyl, morpholinyl, indolinyl, pyrindolinyl, tetrahydroquinolyl, thiazolidinyl and naphthyridyl; optionally substituted with one or more radicals, which may be identical or different, chosen from halogen atoms and alkyl, phenyl and phenylalkyl radicals;
   the said products of formula (Ia) being in all the possible racemic, enantiomeric and diastereoisomeric isomer forms, and also the addition salts with mineral and organic acids or with mineral and organic bases of the said products of formula (Ia).

In the products of formula (I) and subsequently, the terms indicated have the following meanings:
- the term "Hal", "Halo" or halogen denotes fluorine, chlorine, bromine or iodine atoms,
- the term "alkyl radical", "alk", "Alk" or "ALK" denotes a linear or branched radical containing up to 12 carbon atoms, chosen from methyl, ethyl, propyl, isopropyl, butyl, isobutyl, sec-butyl, tert-butyl, pentyl, isopentyl, sec-pentyl, tert-pentyl, neopentyl, hexyl, isohexyl, sec-hexyl, tert-hexyl, heptyl, octyl, nonyl, decyl, undecyl and dodecyl radicals, and also the linear or branched positional isomers thereof.

Mention is made more particularly of alkyl radicals containing up to 6 carbon atoms, and especially methyl, ethyl, propyl, isopropyl, n-butyl, isobutyl, tert-butyl, linear or branched pentyl and linear or branched hexyl radicals.
- the term "alkenyl radical" denotes a linear or branched radical containing up to 12 carbon atoms and preferably 4 carbon atoms, chosen, for example, from the following values: ethenyl or vinyl, propenyl or allyl, 1-propenyl, n-butenyl, i-butenyl, 3-methyl-2-butenyl, n-pentenyl, hexenyl, heptenyl, octenyl, cyclohexylbutenyl and decenyl, and also the linear or branched positional isomers thereof.

Among the alkenyl values that may be mentioned more particularly are the values allyl or butenyl.
- the term "alkynyl radical" denotes a linear or branched radical containing up to 12 carbon atoms and preferably 4 carbon atoms, chosen, for example, from the following values: ethynyl, propynyl or propargyl, butynyl, n-butynyl, i-butynyl, 3-methyl-2-butynyl, pentynyl or hexynyl, and also the linear or branched positional isomers thereof.

Among the alkynyl values that are mentioned more particularly is the propargyl value.
- the term "alkoxy radical" denotes a linear or branched radical containing up to 12 carbon atoms and preferably 6 carbon atoms chosen, for example, from methoxy, ethoxy, propoxy, isopropoxy, linear, secondary or tertiary butoxy, pentoxy, hexoxy and heptoxy radicals, and also the linear or branched positional isomers thereof,
- the term "alkoxycarbonyl radical" or alkyl-O-CO-denotes a linear or branched radical containing up to 12 carbon atoms, in which the alkyl radical has the meaning given above: examples that may be mentioned include methoxycarbonyl and ethoxycarbonyl radicals,
- the term "alkylenedioxy radical" or -O-alkylene-O-denotes a linear or branched radical containing up to 12 carbon atoms, in which the alkylene radical has the meaning given above: examples that may be mentioned include methylenedioxy and ethylenedioxy radicals,
- the term "alkylsulfinyl" or alkyl-SO- denotes a linear or branched radical containing up to 12 carbon atoms, in which the alkyl radical has the meaning given above and preferably contains 4 carbon atoms,
- the term "alkylsulfonyl" or alkyl-SO2- denotes a linear or branched radical containing up to 12 carbon atoms, in which the alkyl radical has the meaning given above and preferably contains 4 carbon atoms,
- the term "alkylsulfonylcarbamoyl" or alkyl-S02-NH-C(=O)- denotes a linear or branched radical containing up to 12 carbon atoms, in which the alkyl radical has the meaning given above and preferably contains 4 carbon atoms,
- the term "alkylthio" or alkyl-S- denotes a linear or branched radical containing up to 12 carbon atoms and especially represents methylthio, ethylthio, isopropylthio and heptylthio radicals,
- the term "cycloalkyl radical" denotes a 3- to 10-membered monocyclic or bicyclic carbocyclic radical and especially denotes cyclopropyl, cyclobutyl, cyclopentyl and cyclohexyl radicals,
- the term "-O-cycloalkyl radical" denotes a radical in which the cycloalkyl radical has the meaning given above,
- the term "cycloalkenyl radical" denotes a 3- to 10-membered monocyclic or bicyclic nonaromatic carbocyclic radical containing at least one double bond, and especially denotes cyclobutenyl, cyclopentenyl and cyclohexenyl radicals,
- the term "cycloalkylalkyl radical" denotes a radical in which cycloalkyl and alkyl are chosen from the values indicated above: this radical thus denotes, for example, cyclopropylmethyl, cyclopentylmethyl, cyclohexylmethyl and cycloheptylmethyl radicals,
- the term "acyl radical" or r-CO- denotes a linear or branched radical containing up to 12 carbon atoms, in which the radical r represents a hydrogen atom or an alkyl, cycloalkyl, cycloalkenyl, cycloalkyl, heterocycloalkyl or aryl radical, these radicals having the values indicated above and being optionally substituted as indicated: examples that are mentioned include the formyl, acetyl, propionyl, butyryl or benzoyl radicals, or alternatively valeryl, hexanoyl, acryloyl, crotonoyl or carbamoyl,
- the term "acyloxy radical" means acyl-O- radicals in which acyl has the meaning given above: examples that are mentioned include acetoxy or propionyloxy radicals,
- the term "acylamino radical" means acyl-NH- radicals in which acyl has the meaning given above,
- the term "aryl radical" denotes unsaturated monocyclic radicals or unsaturated radicals consisting of fused carbocyclic rings. Examples of such aryl radicals that may be mentioned include phenyl or naphthyl radicals.

Mention is made more particularly of the phenyl radical.
- the term "arylalkyl" means radicals resulting from the combination of the optionally substituted alkyl radicals mentioned above and the optionally substituted aryl radicals also mentioned above: examples that are mentioned include benzyl, phenylethyl, 2-phenethyl, triphenylmethyl or naphthalenemethyl radicals,
- the term "heterocyclic radical" denotes a saturated carbocyclic radical (heterocycloalkyl) or unsaturated carbocyclic radical (heteroaryl) which is at most 6-membered, interrupted with one or more hetero atoms, which may be identical or different, chosen from oxygen, nitrogen and sulfur atoms.

Heterocycloalkyl radicals that may especially be mentioned include dioxolane, dioxane, dithiolane, thiooxolane, thiooxane, oxiranyl, oxolanyl, dioxolanyl, piperazinyl, piperidyl, pyrrolidyl, imidazolidinyl, pyrazolidinyl, morpholinyl, or tetrahydrofuryl, tetrahydrothienyl, chromanyl, dihydrobenzofuranyl, indolinyl, piperidyl, perhydropyranyl, pyrindolinyl, tetrahydroquinolyl, tetrahydroisoquinolyl and thioazolidinyl radicals, all these radicals being optionally substituted.

Among the heterocycloalkyl radicals that may especially be mentioned are optionally substituted piperazinyl, optionally substituted piperidyl, optionally substituted pyrrolidinyl, imidazolidinyl, pyrazolidinyl, morpholinyl and thioazolidinyl radicals: mention may also be made more particularly of optionally substituted morpholinyl, pyrrolidyl and piperazinyl radicals;
- the term "heterocycloalkylalkyl radical" means radicals in which the heterocycloalkyl and alkyl residues have the above meanings;
- among the 5-membered heteroaryl radicals that may be mentioned are furyl radicals such as 2-furyl, thienyl radicals such as 2-thienyl and 3-thienyl, and pyrrolyl, diazolyl, thiazolyl, thiadiazolyl, thiatriazolyl, isothiazolyl, oxazolyl, oxadiazolyl, 3- or 4-isoxazolyl, imidazolyl, pyrazolyl and isoxazolyl radicals.

Among the 6-membered heteroaryl radicals that may especially be mentioned are pyridyl radicals such as 2-pyridyl, 3-pyridyl and 4-pyridyl, and pyrimidyl, pyrimidinyl, pyridazinyl, pyrazinyl and tetrazolyl radicals;
- as fused heteroaryl radicals containing at least one hetero atom chosen from sulfur, nitrogen and oxygen, examples that may be mentioned include benzothienyl such as 3-benzothienyl, benzofuryl, benzofuranyl, benzo-pyrrolyl, benzimidazolyl, benzoxazolyl, thionaphthyl, indolyl, purinyl, quinolyl, isoquinolyl and naphthyridinyl.

Among the fused heteroaryl radicals that may be mentioned more particularly are benzothienyl, benzofuranyl, indolyl, quinolyl, benzimidazolyl, benzothiazolyl, furyl, imidazolyl, indolizinyl, isoxazolyl, isoquinolyl, isothiazolyl, oxadiazolyl, pyrazinyl, pyridazinyl, pyrazolyl, pyridyl, pyrimidinyl, pyrrolyl, quinazolinyl, 1.3.4-thiadiazolyl, thiazolyl and thienyl radicals and triazolyl groups, these radicals optionally being substituted as indicated for the heteroaryl radicals;
- the term "cyclic amine" denotes a 3- to 8-membered cycloalkyl radical in which one carbon atom is replaced with a nitrogen atom, the cycloalkyl radical having the meaning given above and also possibly containing one or more other hetero atoms chosen from O, S, SO2, N and NR4 with R1 as defined above; examples of such cyclic amines that may be mentioned include pyrrolidyl, piperidyl, morpholinyl, piperazinyl, indolinyl, pyrindolinyl and tetrahydroquinolyl radicals.

The term "patient" denotes human beings, but also other mammals.

The term "prodrug" denotes a product that may be converted in vivo via metabolic mechanisms (such as hydrolysis) into a product of formula (I). For example, an ester of a product of formula (I) containing a hydroxyl group may be converted by hydrolysis in vivo into its parent molecule. Alternatively, an ester of a product of formula (I) containing a carboxyl group may be converted by in vivo hydrolysis into its parent molecule.

Examples of esters of the products of formula (I) containing a hydroxyl group that may be mentioned include the acetates, citrates, lactates, tartrates, malonates, oxalates, salicylates, propionates, succinates, fumarates, maleates, methylenebis-β-hydroxynaphthoates, gentisates, isethionates, di-p-tolyltartrates, methanesulfonates, ethanesulfonates, benzenesulfonates, p-toluenesulfonates, cyclohexylsulfamates and quinates.

Esters of products of formula (I) that are particularly useful, containing a hydroxyl group, may be prepared from acid residues such as those described by Bundgaard et. al., J. Med. Chem., 1989, 32, page 2503-2507: these esters especially include substituted (aminomethyl)benzoates, dialkylaminomethylbenzoates in which the two alkyl groups may be linked together or may be interrupted with an oxygen atom or with an optionally substituted nitrogen atom, i.e. an alkylated nitrogen atom, or alternatively (morpholinomethyl)benzoates, eg. 3- or 4-(morpholinomethyl)benzoates, and (4-alkyl-piperazin-1-yl)benzoates, eg. 3- or 4-(4-alkylpiperazin-1-yl)benzoates.

The carboxyl radical(s) of the products of formula (I) may be salified or esterified with various groups known to those skilled in the art, among which nonlimiting examples that may be mentioned include the following compounds:
- among the salification compounds, mineral bases such as, for example, one equivalent of sodium, potassium, lithium, calcium, magnesium or ammonium, or organic bases such as, for example, methylamine, propylamine, trimethylamine, diethylamine, triethylamine, N,N-dimethylethanolamine, tris(hydroxymethyl)aminomethane, ethanolamine, pyridine, picoline, dicyclohexylamine, morpholine, benzylamine, procaine, lysine, arginine, histidine or N-methylglucamine,
- among the esterification compounds, alkyl radicals to form alkoxycarbonyl groups such as, for example, methoxycarbonyl, ethoxycarbonyl, tert-butoxycarbonyl or benzyloxycarbonyl, these alkyl radicals possibly being substituted with radicals chosen, for example, from halogen atoms and hydroxyl, alkoxy, acyl, acyloxy, alkylthio, amino or aryl radicals, such as, for example, in chloromethyl, hydroxypropyl, methoxymethyl, propionyloxymethyl, methylthiomethyl, dimethylaminoethyl, benzyl or phenethyl groups.

The term "esterified carboxyl" means, for example, radicals such as alkyloxycarbonyl radicals, for example methoxycarbonyl, ethoxycarbonyl, propoxycarbonyl, butyl or tert-butyloxycarbonyl, cyclobutyloxycarbonyl, cyclopentyloxycarbonyl or cyclohexyloxycarbonyl.

Mention may also be made of radicals formed with readily cleavable ester residues, such as methoxymethyl or ethoxymethyl radicals; acyloxyalkyl radicals such as pivaloyloxymethyl, pivaloyloxyethyl, acetoxymethyl or acetoxyethyl; alkyloxycarbonyloxyalkyl radicals such as methoxycarbonyloxy methyl or ethyl radicals, and isopropyloxycarbonyloxy methyl or ethyl radicals.

A list of such ester radicals may be found, for example, in European patent EP 0 034 536.

The term "amidated carboxyl" means radicals of the type -CONR5R6 as defined above.

The term "alkylamino radical" means linear or branched methylamino, ethylamino, propylamino or butylamino radicals. Alkyl radicals containing up to 4 carbon atoms are preferred, the alkyl radicals possibly being chosen from the alkyl radicals mentioned above.

The term "dialkylamino radical" means, for example, dimethylamino, diethylamino and methylethylamino radicals. As previously, alkyl radicals containing up to 4 carbon atoms, chosen from the list indicated above, are preferred.

The radicals NR5R6 or NR11R12 may also represent a heterocycle which may or may not comprise an additional hetero atom. Mention may be made of pyrrolyl, imidazolyl, indolyl, piperidyl, morpholinyl and piperazinyl radicals. The piperidyl, morpholinyl and piperazinyl radicals are preferred.

The term "salified carboxyl" means the salts formed, for example, with one equivalent of sodium, potassium, lithium, calcium, magnesium or ammonium. Mention may also be made of the salts formed with organic bases such as methylamine, propylamine, trimethylamine, diethylamine and triethylamine. The sodium salt is preferred.

When the products of formula (I) comprise an amino radical that may be salified with an acid, it is clearly understood that these acid salts also form part of the invention. Mention may be made of the salts obtained, for example, with hydrochloric acid or methanesulfonic acid.

The addition salts with mineral or organic acids of the products of formula (I) may be, for example, the salts formed with hydrochloric acid, hydrobromic acid, hydriodic acid, nitric acid, sulfuric acid, phosphoric acid, propionic acid, acetic acid, trifluoroacetic acid, formic acid, benzoic acid, maleic acid, fumaric acid, succinic acid, tartaric acid, citric acid, oxalic acid, glyoxylic acid, aspartic acid, ascorbic acid, alkylmonosulfonic acids such as, for example, methanesulfonic acid, ethanesulfonic acid or propanesulfonic acid, alkyldisulfonic acids such as, for example, methanedisulfonic acid or alpha,beta-ethanedisulfonic acid, arylmonosulfonic acids such as benzenesulfonic acid, and aryldisulfonic acids.

It may be recalled that stereoisomerism may be defined in its broad sense as the isomerism of compounds having the same structural formulae but whose various groups are arranged differently in space, especially such as in monosubstituted cyclohexanes whose substituent may be in an axial or equatorial position, and the various possible rotational conformations of ethane derivatives. However, there is another type of stereoisomerism, due to the different spatial arrangements of fixed substituents, either on double bonds or on rings, which is often referred to as geometrical isomerism or cis-trans isomerism. The term "stereoisomer" is used in the present patent application in its broadest sense and thus relates to all the compounds indicated above.

A subject of the invention is especially the products of formula (I) as defined above, such that p represents the integer 0, the other substituents of the said products of formula (I) having any one of the values defined above.

A subject of the invention is especially the products of formula (I) as defined above, such that p represents the integer 1, the other substituents of the said products of formula (I) having any one of the values defined above.

A subject of the invention is especially the products of formula (I) as defined above, such that p represents the integer 2, the other substituents of the said products of formula (I) having the values defined in the present invention.

A subject of the present invention is especially the products of formula (I) or (Ia) as defined above corresponding to formula (Ib): 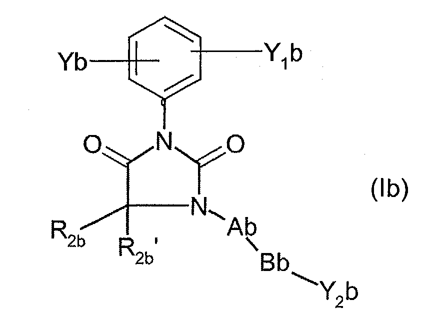
in which
Yb and Y1b, which may be identical or different, are such that:
Y is chosen from:
   alkyl containing 1 to 6 carbon atoms optionally substituted with one or more fluorine atoms and OH;
   3- to 6-membered cycloalkyl optionally substituted with one or more methyl radicals;
   alkylamino;
   dialkylamino, in which the two alkyl residues may optionally form, together with the nitrogen atom to which they are attached, a 5- or 6-membered heterocycle optionally containing one or more other hetero atoms, which may be identical or different, chosen from O, N, Nalkyl and S and optionally substituted with one or more radicals, which may be identical or different, chosen from fluorine atoms and alkyl radicals;
   phenylalkylamino;
   5- to 6-membered phenylmercapto or heteroarylmercapto, optionally substituted with one or more radicals, which may be identical or different, chosen from halogen atoms and alkyl radicals;
   and the other from among Yb and Y1b is chosen from the same values and also from hydrogen; halogen; hydroxyl; oxo; nitro; NR5bR6b; optionally substituted alkyl, alkoxy -S-alkyl and phenyl; optionally substituted pyrazolyl, pyridyl, morpholino, pyrrolidinyl and piperazinyl; free or esterified carboxyl;
   R2b and R2b' represent hydrogen and alkyl, or two substituents R2b and R2b', can form, together with the carbon atom to which they are attached, a cycloalkyl radical containing from 3 to 6 carbon atoms, or form an azetidinyl, pyrrolidinyl or piperidyl radical.
   Ab represents a single bond an alkylene radical;; O; NH; NH-alkyl;
   Bb represents a heterocyclic radical chosen from 3- or 4-pyridyl ; pyrimidinyl;3- or 4- quinolyl ; azaindolyl ; quinazolyl; indazolyl; thiazolyl; imidazolyl; pyrazolyl, furazanyl and isoxazolyl radicals ; these radicals being optionally substituted with one or more radicals chosen from the values of Yb,
   Y2b represents hydrogen; halogen; hydroxyl; alkyl; alkoxy; cycloalkyl; heterocycloalkyl; phenyl; heteroaryl; O-cycloalkyl; S(O)n-alk; S(O)n-cycloalkyl; COOR9; OCOR8; -NR10b-CO-NR5bR6b; NR5R6; CONR5R6; S(O)n-R5R6; NHCOR8 and NH-S(O)nR8; all these radicals being optionally substituted,
   R4b represents a hydrogen atom or an alkyl, cycloalkyl or phenyl radical,
   R5b and R6b, which may be identical or different, are chosen from hydrogen, alkyl, alkenyl, cycloalkyl, heterocycloalkyl, optionally substituted phenyl and heteroaryl or alternatively R5b and R6b form, with the nitrogen atom to which they are attached, a 3- to 10-membered heterocyclic radical containing one or more hetero atoms chosen from O, S, N and optionally substituted NR4b;
   all the above alkyl, alkenyl, alkynyl and alkoxy radicals being linear or branched and containing up to 6 carbon atoms;
   all the above cycloalkyl and heterocycloalkyl radicals containing up to 7 carbon atoms;
   all the above aryl and heteroaryl radicals containing up to 10 carbon atoms;
   all the above optionally substituted radicals being optionally substituted with one or more radicals, which may be identical or different, chosen from halogen, cyano, hydroxyl, alkyl and alkoxy containing 1 to 4 carbon atoms, CF3, nitro, phenyl, carboxyl, free, salified, esterified with an alkyl radical or amidated with a radical NR11bR12b, -C(=O)-R9b, -NR11bR12b or -C(=O)-NR11bR12b ;
   R8b represents alkyl, cycloalkyl, cycloalkylalkyl and phenyl;
   R9b, which may be identical to or different from R8b, represents hydrogen and the values of R8b;
   R11b and R12b, which may be identical or different, represent hydrogen, alkyl, cycloalkyl and phenyl or alternatively R11b and R12b form, with the nitrogen atom to which they are attached, a pyrolidine, piperidinyl, morpholinyl or a piperazinyl radical optionally substituted with an alkyl, phenyl or phenylalkyl radical; the said products of formula (Ib) being in all the possible racemic, enantiomeric and diastereoisomeric isomer forms, and also the addition salts with mineral and organic acids or with mineral and organic bases of the said products of formula (Ib).

A subject of the present invention is especially the products of formula (I), (Ia) or (Ib) as defined above corresponding to formula (Ic): 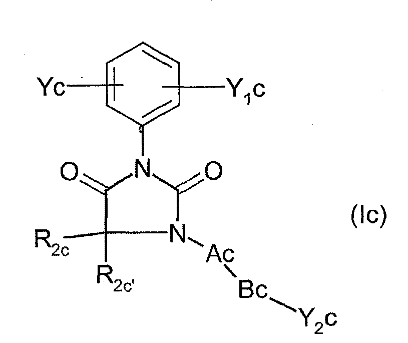
in which Yc and Y1c, which may be identical or different, are such that one from among Yc and Y1c is chosen from alkyl especially such as methyl, ethyl, isopropyl, tert-butyl, sec-butyl, 1,2-dimethylpropyl, 1,1-dimethylpropyl optionally substituted with one or more fluorine atoms and OH; cyclopropyl or cyclobutyl especially such as 1-methylcyclopropyl, 2-methylcyclopropyl, 2,2-dimethylcyclopropyl, cyclobutyl, 2,2,3,3-tetrafluorocyclobutyl; di(C2-C4-alkyl)amino; phenylalkylamino; piperid-1-yl, thiomorpholin-4-yl, morpholin-4-yl, pyrrolidin-1-yl optionally substituted with one or more radicals chosen from fluorine atoms and alkyl radicals; phenylmercapto, optionally substituted with one or more halogen atoms ; and the other from among Yc and Y1c is chosen from hydrogen; halogen; hydroxyl (oxo); nitro; NR5cR6c; optionally substituted alkyl, alkoxy, -S-alkyl and phenyl; optionally substituted pyrazolyl and pyridyl;
R2 and R2c'represent a hydrogen atom, alkyl of form together with the carbon atom bearing them a 3 to 6 membered cycloalkyl ring
Ac represents a single bond, -O- or -CH2;
Bc represents a heterocyclic radical chosen from 3- or 4-pyridyl, pyrimidinyl, , 3- or 4- quinolyl, azaindolyl and quinazolyl, these radicals being optionally substituted with one or more radicals chosen from the values of Y2c, Y2c represents hydrogen; halogen; alkyl; cycloalkyl; hydroxyl; alkoxy; NH2; NHalk; N(alk)2; NH-Phenyl-; NH-Heteroaryl; NH-CO-R5c; NH-CO-heteroaryl; NH-CO-NR5cR6c; and phenyl; all the alkyl, alkoxy, phenyl and heteroaryl radicals being optionally substituted;
R5c and R6c, which may be identical or different, represent hydrogen, alkyl, cycloalkyl and phenyl, which are optionally substituted, or alternatively R5c and R6c form, with the nitrogen atom to which they are attached, a cyclic radical chosen from pyrrolidinyl, piperidyl, piperazinyl, morpholinyl, piperazinyl, indolinyl, pyrindolinyl, tetrahydroquinoline and azetidine radicals; all these radicals being optionally substituted with one or more radicals chosen from alkyl, alkoxy and phenyl;
in all the above radicals, the alkyl, alkoxy and phenyl radicals are optionally substituted with one or more radicals chosen from halogen, OH, alk, Oalk, OCF3, S(O)n-CF3, CF3, NH2, NHAlk and N(alk)2; it beeing understood, that all dialkylamino radicals optionally can form a pyrrolidine, piperidine, morpholine or piperazine ring optionally substituted by one or more alkyl;
the said products of formula (Ic) being in all the possible racemic, enantiomeric and diastereoisomeric isomer forms, and also the addition salts with mineral and organic acids or with mineral and organic bases of the said products of formula (Ic).

In particular, R5c and R6c, which may be identical or different, represent hydrogen, alkyl, cycloalkyl and phenyl, the alkyl and phenyl radicals being optionally substituted, or alternatively R5c and R6c form, with the nitrogen atom to which they are attached, a cyclic radical chosen from pyrrolidinyl, piperidyl, piperazinyl, morpholinyl, piperazinyl and azetidine.

In particular, Bc represents a heterocyclic radical chosen from 3- or 4-pyridyl, 1H-pyrrolo[2,3-b]pyridin-4-yl pyrimidinyl and 3- or 4-quinolyl.

Most particularly, Bc represents 4-pyridyl and 4-quinolyl radicals, optionally substituted with one or more radicals chosen from the values of Y2c.

A subject of the present invention is especially the products of formula (I) as defined above corresponding to formula (Id): 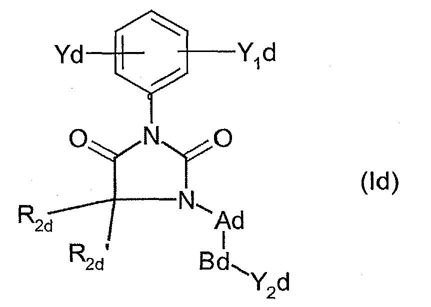
in which
Yd is chosen from the following values: alkyl especially such as methyl, ethyl, isopropyl, tert-butyl, sec-butyl, 1,2-dimethylpropyl, 1,1-dimethylpropyl optionally substituted with one or more fluorine atoms and OH; cyclopropyl or cyclobutyl such as, especially, 1-methylcyclopropyl, 2-methylcyclopropyl, 2,2-dimethylcyclopropyl, cyclobutyl, 2,2,3,3-tetrafluorocyclobutyl; di(C2-C4-alkyl)amino; phenylalkylamino; piperid-1-yl, thiomorpholin-4-yl, morpholin-4-yl or pyrrolidin-1-yl, optionally substituted with one or more radicals chosen from fluorine atoms and alkyl radicals; phenylmercapto, optionally substituted with one or more halogen atoms; and Y1d is chosen from the following values: hydrogen; halogen; OH; oxo; nitro, NH2; free or esterified carboxyl; morpholino; alkyl, alkoxy, S-alkyl and phenyl optionally substituted;
in all these radicals, the alkyl and phenyl radicals are themselves optionally substituted with one or more radicals, which may be identical or different, chosen from halogen atoms and alkyl, alkoxy, OCF3, cyano, amino, alkylamino and dialkylamino radicals, and a phenyl radical, itself optionally substituted with one or more radicals, which may be identical or different, chosen from halogen atoms, and alkyl and alkoxy radicals;
R2d and R2d', which may be identical or different, are chosen from hydrogen or alkyl or form together with the atom bearing them a cyclopropyl or acyclobutyl ring;
Ad represents a single bond or CH2;
Bd represents a quinolyl or pyridyl radical optionally substituted with one or more radicals Y2d chosen from halogen, -OH, alk, -Oalk, -CO2H, -CO2alk, -NH2, NHalk, N(alk)2, -CF3, -OCF3 and phenyl; NH-phenyl; NH-heteroaryl NH-CO-phenyl, NH-CO-heteroaryl; NH-CO-NH-alkyl ; NH-CO-N-dialkyl; NH-CO-NH-phenyl; the alkyl and phenyl radicals being themselves optionally substituted with one or more radicals chosen from halogen atoms and alkyl and alkoxy and dialkylamino radicals; it beeing understood, that all dialkylamino radicals optionally can form a 5- to 7 membered ring, optionally containing one more heteroatom or group from the series N,N-alkyl, O and S;
the said products of formula (Id) being in all the possible racemic, enantiomeric and diastereoisomeric isomer forms, and also the addition salts with mineral and organic acids or with mineral and organic bases of the said products of formula (Id).

In the products of formula (I) as defined above, when Y1d represents optionally substituted alkyl, alkoxy or -S-alkyl, Y1d may especially represent CF3; -O-CF2-CHF2 ; -O-CHF2 ; -O-CH2-CF3 ; -S-CF2-CF2-CF3; -S-Alk-O-Alk ; -S-Alk-OH; -S-Alk-CN ; -S-Alk-heterocycloalkyl.

More particularly, B represents a 4-quinolyl or 4-pyridyl radical optionally substituted with one or more radicals chosen from F, Cl , OH, CH3, CH2CH3, OCH3, NH2, NHAlk and N(alk)2, and phenyl, NH-phenyl; NH-heteroaryl NH-CO-phenyl, NH-CO-heteroaryl; NH-CO-NH-alkyl; NH-CO-NH-dialkyl the alkyl and phenyl radicals being themselves optionally substituted with one or more radicals chosen from halogen atoms and alkyl and alkoxy radicals.

Most particularly, B represents 4-pyridyl and 4-quinolyl radicals substituted with one or two radicals chosen from F, Cl, OH ; NH2 and OCH3.

A subject of the present invention is, most particularly, the products of formula (I) as defined above
in which
Y is chosen from the following values:
alkyl especially such as methyl, ethyl, isopropyl, tert-butyl, sec-butyl, 1,2-dimethylpropyl and 1,1-dimethylpropyl, optionally substituted with one or more fluorine atoms and OH;
di(C2-C4-alkyl)amino;
phenylalkylamino;
piperid-1-yl, thiomorpholin-4-yl, morpholin-4-yl, pyrrolidin-1-yl;
and Y1 is chosen from the following values: hydrogen, halogen, nitro, NH2 and alkyl;
R2 and R2', which may be identical or different, are chosen from hydrogen or alkyl;
A represents CH2;
B represents a quinolyl or pyridyl radical;
the said products of formula (I) being in all the possible racemic, enantiomeric and diastereoisomeric isomer forms, and also the addition salts with mineral and organic acids or with mineral and organic bases of the said products of formula (I).

More particularly, R2 and R2', which may be identical or different, are chosen from hydrogen or methyl and ethyl.

Among the preferred products of the invention, mention may be made more specifically of the products of formula (I) as defined above, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3-[4-(4-Fluoro-phenylsulfanyl)-phenyl]-5,5-dimethyl-1-pyridin-4-ylmethyl-imidazolidine-2,4-dione trifluoroacetate
- 3-(4-Isopropyl-3-nitro-phenyl)-5,5-dimethyl-1-pyridin-4-ylmethyl-imidazolidine-2,4-dione
- 3-(3-Amino-4-isopropyl-phenyl)-5,5-dimethyl-1-pyridin-4-ylmethyl-imidazolidine-2,4-dione

the said products of formula (I) being in all the possible racemic, enantiomeric and diastereoisomeric isomer forms, and also the addition salts with mineral and organic acids or with mineral and organic bases of the said products of formula (I).

Among the preferred products of the invention, mention may be made more specifically of the products of formula (I) as defined above, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3-[4-(4-Fluoro-phenylsulfanyl)-phenyl]-5,5-dimethyl-1-pyridin-4-ylmethyl-imidazolidine-2,4-dione trifluoroacetate

the said products of formula (I) being in all the possible racemic, enantiomeric and diastereoisomeric isomer forms, and also the addition salts with mineral and organic acids or with mineral and organic bases of the said products of formula (I).

The compounds of the general formula **I** can be prepared by initially converting an aniline of the general formula **1** in which Y*'* and Y1*'* have the meanings stated for Y and Y1 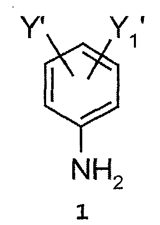
by reaction with phosgene, diphosgene or triphosgene, or by activation with carbonyldiimidazole or a reagent of a similar type, into a reactive intermediate such as, for example, the isocyanate 2 or the carbonylimidazole derivative 3.

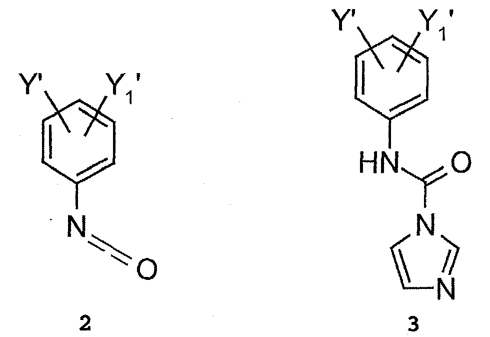

These reactive derivatives are prepared in an inert organic solvent such as, for example, toluene, 1,2-dichloroethane or THF, at a temperature between -20°C and the reflux temperature of the particular solvent. Preferred solvents are toluene and 1,2-dichlorethane, and preferred reaction temperatures are from -20 to +5°C during the addition and subsequent heating to the reflux temperature to complete the reaction. The reaction can be assisted by addition of a base, but is preferably carried out without addition of base.

The reactive derivatives of the general formula **2** can be isolated, but the intermediates are preferably used without further purification, where appropriate after replacement of the solvent, directly for further reaction.

Reaction of the intermediates with a structural unit of the general formula **4** in which Z is COOR or CN is carried out in an inert organic solvent such as, for example, toluene, chlorobenzene, THF, dioxane or ethyl acetate, at a temperature between room temperature and the reflux temperature of the solvent. The reaction can be assisted by addition of a base such as, for example, triethylamine or potassium tert-butoxide, but is preferably carried out without addition of base. The initial linkage of the reactive intermediates **2** or **3** with the amino derivative of the general formula **4** and the subsequent ring closure to form the central heterocycle is preferably carried out in one step.

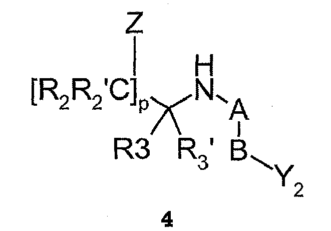

However, it is also possible alternatively to form the central heterocycle in a second step by heating the open-chain intermediate of the general formula **5** in a higher-boiling solvent or in aqueous mineral acids.

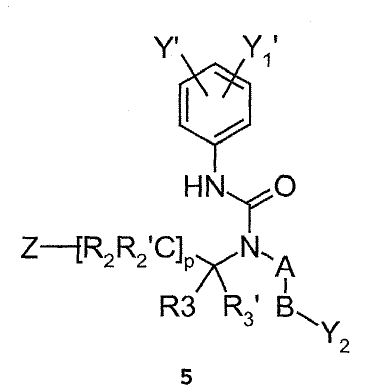

In the case where Z is CN, the products of the general formula **Ia** (R1=N) are obtained and can be converted through the action of aqueous mineral acid into compounds of the general formula **Ib**.

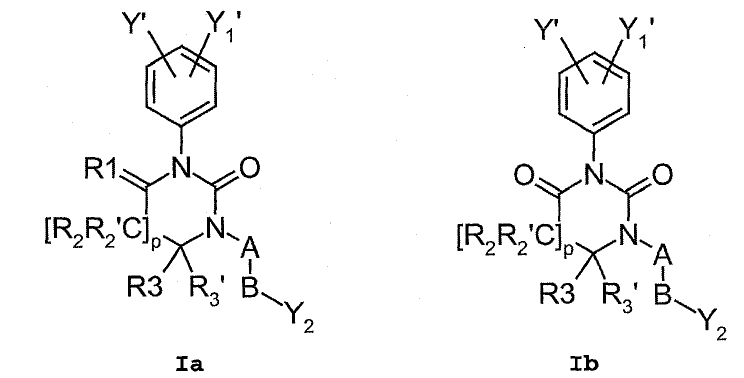

The compounds of the general formulae **Ia** and **Ib** prepared in this way, in which the variables have the meanings stated above for the compound of the general formula **I**, can be converted by derivatization reactions known to the skilled worker into compounds of the general formula **I**.

An alternative approach to compounds of the general formula **I** is provided by reaction of the structural unit **4** with carbonyldiimidazole, phosgene, diphosgene or triphosgene to give a reactive intermediate. The reaction is preferably carried out with carbonyldiimidazole in an inert organic solvent such as, for example, toluene, 1,2-dichloroethane or THF at a temperature between -20°C and RT. THF is the particularly preferred solvent. The intermediates such as, for example, the derivatives of the general formula **6** (reaction with carbonyldiimidazole) are then reacted in a solvent such as, for example, DMF, toluene, 1,2-dichloroethane or THF with an aniline of the general formula 1. The reaction is preferably carried out at a temperature between RT and the boiling point of the solvent. Open-chain intermediates are preferably cyclized directly to compounds of the general formula **Ia** and **b**, which can be converted by further derivatization reactions known to the skilled worker into compounds of the general formula **I**.

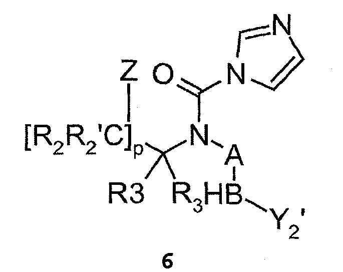

The compounds of the general formula **Ia** can additionally be obtained by reacting compounds of the general formulae **2** and **3** with a structural unit of the general formula **7.**

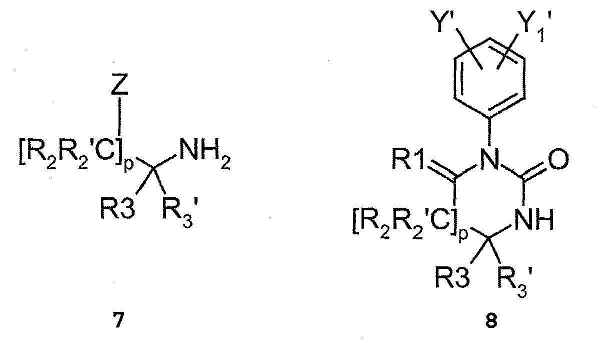

The resulting derivatives of the general formula **8** can then be converted by the action of a halide Hal-A'-B'-Y₂' or a related reagent of similar reactivity in which A', B' and Y₂*'* have the meanings mentioned for A, B and Y₂, and which are obtainable by conventional processes known to the skilled worker, into compounds of the general formula **Ia**.

The reactions are preferably carried out in an organic solvent such as, for example, dimethylformamide, N-methylpyrrolidone, ethyl acetate or acetone in the presence of a base such as, for example, potassium carbonate, caesium carbonate, sodium hydride or potassium tert-butoxide. Dimethylformamide and caesium carbonate are preferably used.

All reactions for the synthesis of the compounds of the formula (I) are per se well-known to the skilled person and can be carried out under standard conditions according to or analogously to procedures described in the literature, for example in Houben-Weyl, Methoden der Organischen Chemie (Methods of Organic Chemistry), Thieme-Verlag, Stuttgart, or Organic Reactions, John Wiley & Sons, New York.

It may be noted that, during or after the process, some intermediates compounds or some compounds of formula (I) may be transformed to obtain some (or other) compounds of formula (I) and for that, to obtain products or other products of formula (I), these products may be subjected if desired, and necessary, to one or more of the following conversion reactions, in any order:
a) a reaction for esterification of an acid function,
b) a reaction for saponification of an ester function to an acid function,
c) a reaction for oxidation of an alkylthio group to the corresponding sulfoxide or sulfone group,
d) a reaction for conversion of a ketone function to an oxime function,
e) a reaction for reducing a free or esterified carboxyl function to an alcohol function,
f) a reaction for conversion of an alkoxy function to a hydroxyl function, or alternatively of a hydroxyl function to an alkoxy function,
g) a reaction for oxidation of an alcohol function to an aldehyde, acid or ketone function,
h) a reaction for conversion of a nitrile radical to a tetrazolyl,
i) a reaction for reduction of nitro compounds to amino compounds,
j) a reaction for removal of the protecting groups that may be borne by the protected reactive functions,
k) a reaction for salification with a mineral or organic acid or with a base to obtain the corresponding salt,
l) a reaction for resolution of the racemic forms to resolved products,

said products of formula (I) thus obtained being in any possible racemic, enantiomeric or diastereoisomeric isomer form.

It may be noted that such reactions for converting substituents into other substituents may also be performed on the starting materials, and also on the intermediates as defined above before continuing the synthesis according to the reactions indicated in the process described above.

The various reactive functions that may be borne by certain compounds of the reactions defined above may, if necessary, be protected: these are, for example, hydroxyl, acyl, free carboxyl or amino and monoalkylamino radicals, which may be protected with the appropriate protecting groups.

The following nonexhaustive list of examples of protection of reactive functions may be mentioned:
- the hydroxyl groups may be protected, for example, with alkyl radicals such as tert-butyl, trimethylsilyl, tert-butyldimethylsilyl, methoxymethyl, tetrahydropyranyl, benzyl or acetyl,
- the amino groups may be protected, for example, with acetyl, trityl, benzyl, tert-butoxycarbonyl, benzyloxycarbonyl, phthalimido radicals or other radicals known in peptide chemistry,
- the acyl groups such as the formyl group may be protected, for example, in the form of cyclic or noncyclic ketals or thioketals such as dimethyl or diethylketal or ethylene dioxyketal, or diethylthioketal or ethylenedithioketal,
- the acid functions of the products described above may be, if desired, amidated with a primary or secondary amine, for example in methylene chloride in the presence, for example, of 1-ethyl-3-(dimethylaminopropyl)carbodiimide hydrochloride at room temperature,
- the acid functions may be protected, for example, in the form of esters formed with readily cleavable esters such as benzyl esters or tert-butyl esters, or esters known in peptide chemistry.

These reactions a) to k) indicated above may be performed, for example, as indicated below.
a) The products described above may, if desired, undergo, on the possible carboxyl functions, esterification reactions that may be performed according to the usual methods known to those skilled in the art.
b) The possible conversions of ester functions into an acid function of the products described above may be, if desired, performed under the usual conditions known to those skilled in the art, especially by acid or alkaline hydrolysis, for example with sodium hydroxide or potassium hydroxide in alcoholic medium such as, for example, in methanol, or alternatively with hydrochloric acid or sulfuric acid.
c) The possible alkylthio groups in the products described above, in which the alkyl radical is optionally substituted with one or more halogen atoms, especially fluorine, may, if desired, be converted into the corresponding sulfoxide or sulfone functions under the usual conditions known to those skilled in the art such as, for example, with peracids such as, for example, peracetic acid or meta-chloroperbenzoic acid, or alternatively with ozone, oxone or sodium periodate in a solvent such as, for example, methylene chloride or dioxane at room temperature.

The production of the sulfoxide function may be promoted with an equimolar mixture of the product containing an alkylthio group and the reagent such as, especially, a peracid.

The production of the sulfone function may be promoted with a mixture of the product containing an alkylthio group with an excess of the reagent such as, especially, a peracid.
d) The reaction for conversion of a ketone function into an oxime may be performed under the usual conditions known to those skilled in the art, such as, especially, a reaction in the presence of an optionally O-substituted hydroxylamine in an alcohol such as, for example, ethanol, at room temperature or with heating.
e) The possible free or esterified carboxyl functions of the products described above may be, if desired, reduced to an alcohol function by the methods known to those skilled in the art: the possible esterified carboxyl functions may be, if desired, reduced to an alcohol function by the methods known to those skilled in the art and especially with lithium aluminum hydride in a solvent such as, for example, tetrahydrofuran or dioxane or ethyl ether.

The possible free carboxyl functions of the products described above may be, if desired, reduced to an alcohol function especially with boron hydride.
f) The possible alkoxy functions such as, especially, methoxy, in the products described above, may be, if desired, converted into a hydroxyl function under the usual conditions known to those skilled in the art, for example with boron tribromide in a solvent such as, for example, methylene chloride, with pyridine hydrobromide or hydrochloride or with hydrobromic acid or hydrochloric acid in water or trifluoroacetic acid at reflux.
g) The possible alcohol functions of the products described above may be, if desired, converted into an aldehyde or acid function by oxidation under the usual conditions known to those skilled in the art, such as, for example, by the action of manganese oxide to obtain the aldehydes, or of Jones' reagent to access the acids.
h) The possible nitrile functions of the products described above may be, if desired, converted into tetrazolyl under the usual conditions known to those skilled in the art, such as, for example, by cycloaddition of a metal azide such as, for example, sodium azide or a trialkyltin azide on the nitrile function, as indicated in the method described in the article referenced as follows:
J. Organometallic Chemistry., 33, 337 (1971) KOZIMA S. et al.

It may be noted that the reaction for conversion of a carbamate into urea and especially of a sulfonylcarbamate into sulfonylurea may be performed, for example, at the reflux point of a solvent such as, for example, toluene, in the presence of the appropriate amine.

It is understood that the reactions described above may be performed as indicated or alternatively, where appropriate, according to other common methods known to those skilled in the art.
i) The removal of protecting groups such as, for example, those indicated above may be performed under the usual conditions known to those skilled in the art, especially via an acid hydrolysis performed with an acid such as hydrochloric acid, benzenesulfonic acid or para-toluenesulfonic acid, formic acid or trifluoroacetic acid, or alternatively via a catalytic hydrogenation.

The phthalimido group may be removed with hydrazine.

A list of various protecting groups that may be used will be found, for example, in patent BF 2 499 995.
j) The products described above may, if desired, be subjected to salification reactions, for example with a mineral or organic acid or with a mineral or organic base according to the usual methods known to those skilled in the art.
k) The possible optically active forms of the products described above may be prepared by resolving the racemic mixtures according to the usual methods known to those skilled in the art.

The possible reactive functions are hydroxyl or amino functions. Usual protecting groups are used to protect these functions. Examples that may be mentioned include the following protecting groups for the amino radical: tert-butyl, tert-amyl, trichloroacetyl, chloroacetyl, benzhydryl, trityl, formyl, benzyloxycarbonyl.

Protecting groups for the hydroxyl radical that may be mentioned include radicals such as formyl, chloroacetyl, tetrahydropyranyl, trimethylsilyl and tert-butyldimethylsilyl.

It is clearly understood that the above list is not limiting and that other protecting groups, which are known, for example, in peptide chemistry, may be used. A list of such protecting groups is found, for example, in French patent 2 499 995, the content of which is incorporated herein by reference.

The possible reactions for removal of the protecting groups are performed as indicated in said patent 2 499 995. The preferred method of removal is acid hydrolysis with acids chosen from hydrochloric acid, benzenesulfonic acid or para-toluenesulfonic acid, formic acid or trifluoroacetic acid. Hydrochloric acid is preferred.

The possible reaction for hydrolysis of the >C=NH group to a ketone group is also preferably performed using an acid such as aqueous hydrochloric acid, for example at reflux.

An example of removal of the tert-butyldimethylsilyl group using hydrochloric acid is given below in the examples.
- The possible esterification of a free OH radical is performed under standard conditions. An acid or a functional derivative, for example an anhydride such as acetic anhydride in the presence of a base such as pyridine, may be used, for example.

The possible esterification or salification of a COOH group is performed under the standard conditions known to those skilled in the art.
- The possible amidation of a COOH radical is performed under standard conditions. A primary or secondary amine may be used on a functional derivative of the acid, for example a symmetrical or mixed anhydride.

The products of formula (I) according to the present invention may be prepared by application or adaptation of known methods and especially of the methods described in the literature such as, for example, those described by R.C. Larock in: Comprehensive Organic Transformations, VCH publishers, 1989.

In the reactions described below, it may be necessary to protect reactive functional groups such as, for example, hydroxyl, amino, imino, thio or carboxyl groups, when these groups are desired in the final product but when their participation is not desired in the reactions for synthesizing the products of formula (I). Conventional protecting groups may be used in accordance with the usual standard practices, for instance those described, for example, by T.W. Greene and P.G.M. Wuts in "Protective Groups in Organic Chemistry" John Wiley and Sons, 1991.

The anilines of the general formula **1** are in some cases commercially available or are described in the literature or can be obtained from derivatives disclosed in the literature by transformations known to a person skilled in the art. The precursors **4** can be obtained for example by reductive amination of aldehydes of the general formula OHC-A" -B'-Y₂*'*, which are commercially available or are prepared by conventional processes, with amino acid derivatives or amino nitriles of the general formula **7**.

The products of the present invention are endowed with advantageous pharmacological properties: it has been found that they especially have inhibitory properties on protein kinases.

Among these protein kinases, mention is made especially of IGF1R.

Mention is also made of FAK. Mention is also made of AKT.

These properties thus make products of general formula (I) of the present invention usable as medicinal products for treating malignant tumours.

The products of formula (I) may also be used in the veterinary field.

A subject of the invention is thus the use, as medicinal products, of the pharmaceutically acceptable products of general formula (I).

A subject of the invention is particularly the use, as medicinal products, of the products, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3-[4-(4-Fluoro-phenylsulfanyl)-phenyl]-5,5-dimethyl-1-pyridin-4-ylmethyl-imidazolidine-2,4-dione trifluoroacetate
- 3-(4-Isopropyl-3-nitro-phenyl)-5,5-dimethyl-1-pyridin-4-ylmethyl-imidazolidine-2,4-dione
- 3-(3-Amino-4-isopropyl-phenyl)-5,5-dimethyl-1-pyridin-4-ylmethyl-imidazolidine-2,4-dione

the said products of formula (I) being in all the possible racemic, enantiomeric and diastereoisomeric isomer forms, and also the pharmaceutically acceptable addition salts with mineral and organic acids or with mineral and organic bases of the said products of formula (I) .

A subject of the invention is particularly the use, as medicinal products, of the products, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3- [4- (4-Fluoro-phenylsulfanyl) -phenyl] -5, 5-dimethyl-1-pyridin-4-ylmethyl-imidazolidine-2,4-dione trifluoroacetate

the said products of formula (I) being in all the possible racemic, enantiomeric and diastereoisomeric isomer forms, and also the pharmaceutically acceptable addition salts with mineral and organic acids or with mineral and organic bases of the said products of formula (I) .

The products may be administered via the parenteral, buccal, perlingual, rectal or topical route.

A subject of the invention is also pharmaceutical compositions, characterized in that they contain, as active principle, at least one of the medicinal products of general formula (I).

These compositions may be in the form of injectable solutions or suspensions, tablets, coated tablets, capsules, syrups, suppositories, creams, ointments and lotions. These pharmaceutical forms are prepared according to the usual methods. The active principle may be incorporated into excipients usually used in these compositions, such as aqueous or nonaqueous vehicles, talc, gum arabic, lactose, starch, magnesium stearate, cocoa butter, fatty substances of animal or plant origin, paraffin derivatives, glycols, various wetting, dispersing or emulsifying agents, and preserving agents.

The usual dose, which varies according to the individual treated and the complaint under consideration, may be, for example, from 10 mg to 500 mg per day orally in man.

The present invention thus relates to the use of products of formula (I) as defined above or of pharmaceutically acceptable salts of said products of formula (I) for the preparation of medicinal products for inhibiting the activity of protein kinases and especially of a protein kinase.

The present invention thus relates to the use of products of formula (I) as defined above or of pharmaceutically acceptable salts of said products of formula (I) in which the protein kinase is a protein tyrosine kinase.

The present invention thus relates to the use of products of formula (I) as defined above or of pharmaceutically acceptable salts of said products of formula (I) in which the protein kinase is chosen from the following group: IGF1, Raf, EGF, PDGF, VEGF, Tie2, KDR, Fltl-3, FAK, Src, Abl, cKit, cdk1-9, Aurora1-2, cdc7, Akt, Pdk, S6K, Jnk, IR, FLK-1, FGFR1, FGFR2, FGFR3, FGFR4, FGFR5, PLK, Pyk2, CDK7, CDK2 et EGFR.

Such protein kinase is chosen more especially in the following group: IGF1, cdc7, Auroral-2, Src, Jnk, FAK, KDR, IR, Tie2, CDK7, CDK2 et EGFR.

The present invention thus relates particularly to the use of products of formula (I) as defined above or of pharmaceutically acceptable salts of said products of formula (I) in which the protein kinase is IGF1R.

The present invention also relates to the use of products of formula (I) as defined above or of pharmaceutically acceptable salts of said products of formula (I) in which the protein kinase is FAK.

The present invention also relates to the use of products of formula (I) as defined above or of pharmaceutically acceptable salts of said products of formula (I) in which the protein kinase is AKT.

The present invention also relates to the use of products of formula (I) as defined above or of pharmaceutically acceptable salts of said products of formula (I) in which the protein kinase is in a cell culture, and also to this use in a mammal.

The present invention thus relates to the use of products of formula (I) as defined above or of pharmaceutically acceptable salts of said products of formula (I) for the preparation of a medicinal product for preventing or treating a disease characterized by deregulation of the activity of a protein kinase and especially such a disease in a mammal.

The present invention relates to the use of products of formula (I) as defined above or of pharmaceutically acceptable salts of said products of formula (I) for the preparation of a medicinal product for preventing or treating a disease belonging to the following group: disorders of blood vessel proliferation, fibrotic disorders, disorders of mesangial cell proliferation, acromegaly, metabolic disorders, allergies, asthma, Crohn's disease, thrombosis, diseases of the nervous system, retinopathy, psoriasis, rheumatoid arthritis, diabetes, muscle degeneration, aging, age related macula degeneration, oncology diseases and cancer.

The present invention thus relates to the use of products of formula (I) as defined above or of pharmaceutically acceptable salts of said products of formula (I) for the preparation of a medicinal product for treating oncology diseases.

The present invention relates particularly to the use of products of formula (I) as defined above or of pharmaceutically acceptable salts of said products of formula (I) for the preparation of a medicinal product for treating cancers.

Among these cancers, the present invention is most particularly of interest in the treatment of solid tumours and the treatment of cancers that are resistant to cytotoxic agents.

Among these cancers, the present invention relates most particularly to the treatment of breast cancer, stomach cancer, cancer of the colon, lung cancer, cancer of the ovaries, cancer of the uterus, brain cancer, cancer of the kidney, cancer of the larynx, cancer of the lymphatic system, cancer of the thyroid, cancer of the urogenital tract, cancer of the tract including the seminal vesicle and prostate, bone cancer, cancer of the pancreas and melanomas.

The present invention is even more particularly of interest in treating breast cancer, cancer of the colon and lung cancer.

The present invention also relates to the use of products of formula (I) as defined above or of pharmaceutically acceptable salts of said products of formula (I) for the preparation of a medicinal product for cancer chemotherapy.

As medicinal products according to the present invention for cancer chemotherapy, the products of formula (I) according to the present invention may be used alone or in combination with chemotherapy or radiotherapy or alternatively in combination with other therapeutic agents.

The present invention thus relates especially to the pharmaceutical compositions as defined above, also containing active principles of other chemotherapy medicinal products for combating cancer.

Such therapeutic agents may be commonly used antitumour agents.

As examples of known inhibitors of protein kinases, mention may be made especially of butyrolactone, flavopiridol, 2-(2-hydroxyethylamino)-6-benzylamino-9-methylpurine, olomucine, Glivec and Iressa.

The products of formula (I) according to the present invention may thus also be advantageously used in combination with antiproliferative agents: as examples of such antiproliferative agents, but without, however, being limited to this list, mention may be made of aromatase inhibitors, antiestrogens, the topoisomerase I inhibitors, the topoisomerase II inhibitors, microtubule-active agents, alkylating agents, histone deacetylase inhibitors, farnesyl transferase inhibitors, COX-2 inhibitors, MMP inhibitors, mTOR inhibitors, antineoplastic antimetabolites, platinum compounds, compounds that reduce the activity of protein kinases and also anti-angiogenic compounds, gonadorelin agonists, antiandrogens, bengamides, biphosphonates and trastuzumab.

Examples that may thus be mentioned include anti-microtubule agents, for instance taxoids, vinca alkaloids, alkylating agents such as cyclophosphamide, DNA-intercalating agents, for instance cis-platinum, agents that are interactive on topoisomerase, for instance camptothecin and derivatives, anthracyclines, for instance adriamycin, antimetabolites, for instance 5-fluorouracil and derivatives, and the like.

The present invention thus relates to products of formula (I) as protein kinase inhibitors, said products of formula (I) being in any possible racemic, enantiomeric or diastereoisomeric isomer form, and also the addition salts of said products of formula (I) with pharmaceutically acceptable mineral and organic acids or with pharmaceutically acceptable mineral and organic bases, and also the prodrugs thereof.

The present invention relates particularly to products of formula (I) as defined above as IGF1R inhibitors.

The present invention also relates to products of formula (I) as defined above as FAK inhibitors.

The present invention also relates to products of formula (I) as defined above as AKT inhibitors.

The present invention relates more particularly to the products of formula (IA) as defined above as IGF1R inhibitors.

The examples whose preparation follows are thus products of formula (I) as defined above and illustrate the present invention without, however, limiting it.

### Example 1 : 3-(4-Dimethylamino-phenyl)-5-methyl-1-pyridin-4-ylmethyl-imidazolidine-2,4-dione trifluoroacetate

2 g (1.02 mmol) of Polystyrene AM RAM (Rink resin) (0.51 mmol/g) are suspended in 20 ml of DMF in a 50 ml syringe fitted with a sinter. After agitation for 10 minutes, the DMF is filtered and replaced with 10 ml of a 50% solution of piperidine in DMF. After agitation for one hour at room temperature, the solution is filtered and the resin washed successively with 3 ' 10 ml of DMF, 2 ' 10 ml of methanol and 3 ' 10 ml of DMF. A solution of 0.94 g of Fmoc-Ala(OH) (3 mmol), 0.41 g of HOBt (3 mmol) and 0 . 4 8 ml of DIC (3 mmol) in 10 ml of DMF is added to the resin. The syringe is agitated overnight at room temperature and the resin is then washed successively with 5 ' 10 ml of DMF, 3 ' 10 ml of MeOH and 5 ' 10 ml of DCM. Next, 10 ml of a 50% solution of piperidine in DMF are introduced into the syringe. After agitation for 1 hour, the solution is filtered and the resin washed with 5 ' 10 ml of DMF, 2 ' 10 ml of MeOH, 3 ' 10 ml of DCM and 3 ' 10 ml of THF. Next, a solution of 0.48 ml of pyridine-4-carboxaldehyde (5 mmol) in 10 ml of a 50/50 THF/TEOF mixture is added to 2.1 g (1 mmol) of the resin.

After agitation overnight at room temperature, the solution is filtered and the resin washed with 10' 10 ml of THF. 1.26 g of sodium cyanoborohydride (10 mmoles)in a mixture of 3 ml of MeOH, 7 ml of dichloroethane and 0.2 ml of acetic acid is then added to the resin. The resin is agitated overnight and then, after filtration, washed with 10 *'* 10 ml of DCM, 3 *'* 10 ml of MeOH and 5 *'* 10 ml of DCM. In parallel, a solution of 0.563 g of 4-dimethyl-aniline (0.25 mmol) is treated with 0.025 g of triphosgene (0.083 mmol), followed by 0.023 ml of pyridine (0.25 mmol) at 0°C under nitrogen. After the temperature has warmed gradually to room temperature, the reaction is stirred for 1 hour and a further 0.23 ml of pyridine in 1 ml of DMF is added to the mixture. The solution obtained is transferred into a syringe containing 100 mg of the previous resin (0.1 mmol. The syringe is shaken 2 hours. The mixture is then filtered and the resin washed with 5' 2 ml of DCM, 3 ' 2 ml of MeOH and 2 ' 10 ml of DCM. Finally, the resin is treated with 1 ml of a 90% solution of trifluoroacetic acid in water. The mixture is agitated for 2 hours and then filtered. The resin is washed with 1 ml of MeOH, followed by 1 ml of DCM. The combined filtrates are evaporated under vacuum. The crude product is directly purified by preparative LC-MS giving 12.8 mg (overall yield = 60%) of expected product are isolated in the form of a white solid.
MS(ES⁺): m/e = 325
HPLC Retention time [min] = 2.77
(YMC basic S5 column; 2 85% ACN/H2O gradient over 7 min)

### Example 2 : 3-(4-tert-Butyl-phenyl)-5,5-dimethyl-1-quinolin-4-ylmethyl-imidazolidine-2,4- dione

To a suspension of 497 mg triphosgene in 3 ml toluene at 100°C a solution of 250 mg 4-tert-butyl-phenylamine and 0.55 ml ethyl-diisopropyl-amine in 2 ml toluene was slowly added and the resulting mixture was stirred for 4 hours at 100°C. After cooling to room temperature the yellow oil that separated was directly subjected to the subsequent reaction without further purification. The oil was dissolved in 2 ml tetrahydrofuran and 221 mg 2-methyl-2-[(quinolin-4-ylmethyl)-amino]-propionic acid methyl ester were added. Afterwards the reaction mixture was heated for 12 hours at 90°C. After removal of the solvent under reduced pressure the residue was purified by flash chromatography on silica gel with a dichloromethane/ methanol gradient. The crude product was purified in addition by preparative HPLC (C18 reverse phase column, elution with a water/ acetonitrile gradient with 0.1% trifluoroacetic acid). Lyophilization of the solution yielded a white solid. Yield: 12.6 mg
MS(ES⁺): m/e = 402
1H-NMR (500 MHz,DMSO/TMS): d = 8.95 (d, 1H); 8.33 (d, 1H); 8.13 (d, 1H); 7.89 (t, 1H); 7.76 (t, 1H); 7.72 (d, 1H); 7.53 (d, 2H); 7.40 (d, 2H); 5.20 (s, 2H); 1.45 (s ,6H); 1.32 (s, 9H)

### Example 3 : 3-(4-Isopropyl-phenyl)-5,5-dimethyl-1-quinolin-4-ylmethyl-imidazolidine-2,4- dione

To a suspension of 548 mg triphosgene in 3 ml toluene at 100°C a solution of 250 mg 4-isopropyl-phenylamine and 0.61 ml ethyl-diisopropyl-amine in 2 ml toluene was slowly added and the resulting mixture was stirred for 4 hours at 100°C. After cooling to room temperature the mixture was directly subjected to the subsequent reaction. 240 mg 2-methyl-2-[(quinolin-4-ylmethyl)-amino]-propionic acid methyl ester were added and the resulting mixture was stirred for further 12 hours at 90°C. After cooling to room temperature the precipitated solid was collected and dissolved in a mixture of 2 ml acetonitrile and 0.25 ml water. After filtration the clear solution was purified by preparative HPLC (C18 reverse phase column, elution with a water/ acetonitrile gradient with 0.1% trifluoroacetic acid). Lyophilization of the fractions containing the product yielded a white solid. Yield: 49.8 mg
MS(ES⁺): m/e = 388
1H-NMR (500 MHz,DMSO/TMS): d = 8.96 (d, 1H) ; 8.35 (d, 1H) ; 8.13 (d, 1H) ; 7.92 (t, 1H) ; 7.78 (t, 1H) ; 7.75 (d, 1H) ; 7.39 (s, 4H) ; 5.20 (s, 2H) ; 2.96 (m, 1H) ; 1.45 (s ,6H) ; 1. 24 (d, 6H)

### Example 4a : 4-isopropyl-3-nitroaniline

25 g (185 mmol) 4-isopropyl-aniline were dissolved in to 260 ml conc. sulfuric acid and treated at -5 °C with 17.9 g nitric acid (65%). After stirring the mixture at 0°C for 0.5h, the reaction mixture was poured on ice water, the precipitating product collected via filtration and dried. The resulting 23.8 g product were essentially pure and were used for the subsequent step without further purification.
MS(ES⁺): m/e = 181
LC/MS Retention time [min] = 1.96

### Example 4 : 3-(4-Isopropyl-3-nitro-phenyl)-5,5-dimethyl-1-pyridin-4-ylmethyl-imidazolidine-2,4-dione

6.04 g (30 mmol) diphosgene in 50 ml 1,2-dichlorethane were treated at -20°C with a solution of 2 g (11.1 mmol) 3-nitro-4-isopropyl-aniline in 20 ml 1,2-dichlorethane. The mixture was allowed to come to room temperature and then was heated to 50 °C for 5h. After standing over night, the solvent was evaporated and the residual oil was taken up in 40m1 THF. 3.93 g (18.7 mmol) 2-methyl-2-[(pyridin-4-ylmethyl)-amino]-propionic acid methyl ester in 30 ml THF were added and the mixture was refluxed for 6h. The solvent was evaporated and the residue taken up in ethylacetate and washed with water. THe organic phase was dried, the solvent evaporated and the residue was purified by preparative HPLC. (C18 reversed phase column, elution with a water (0.1% trifluoracetic acid) /acetonitrile gradient) Yield: 2.7 g.
MS(ES⁺): m/e = 383
HPLC Retention time [min] = 1.67

### Example 5 : 3-(3-Amino-4-isopropyl-phenyl)-5,5-dimethyl-1-pyridin-4-ylmethyl-imidazolidine-2,4-dione

2.64 g (6.90 mmol) 5,5-dimethyl-3-(3-nitro-4-isopropylphenyl)-1-pyridin-4-ylmethyl-imidazolidine-2,4-dione were dissolved in 50 ml acetic acid. 7.22 g zinc dust were added in portions while temperature was kept below 45 °C. The mixture was stirred for 1h at RT, then diluted with 100 ml water, made alkaline with 6N sodium hydroxide solution and extracted with methylene chloride. Separating zinc salts were removed by filtration over cellite. Evaporation of the combined organic phases yielded 1.53 g product that were used without further purification.
MS(ES⁺): m/e = 353
HPLC Retention time [min] = 0.91

### Example 6 : 5,5-Dimethyl-3-(4-phenylamino-phenyl)-1-pyridin-4-ylmethyl-imidazolidine-2,4-dione trifluoroacetate

5,1 g (25 mmol) 2-methyl-2-[(pyridin-4-ylmethyl)-amino]-propionic acid methyl ester were dissolved in 102 ml tetrahydrofurane and treated at 0°C with 4.46 g (27.5 mmol) carbonyldiimidazole. The mixture was stirred for 15 min at 0°C and 1h at RT. A 2ml aliquot of this solution was given to (0.5mmol) of the appropriate aniline, dissolved in 1 ml DMF, and stirred at 50 °C for 15 h. Then the reaction mixture was filtered and the sovent was evapotated. The residue was taken up in 20 ml ethylacetate and washed with 20 ml 5% NaHCO3 solution and 20 ml 5% NaCl solution. The phases were separated, the organic phase dried of Chromabond XTR and the solvent evaporated. The raw product was purified by preparative HPLC (C18 reverse phase column, elution with a water/acetonitrile gradient with 0.1% trifluoracetic acid) Yield: 7.7 mg
MS(ES⁺): m/e = 387
HPLC Retention time [min] = 1.51

The following compounds were prepared as described for example 6.

### Example 7 : 3-(4-Diethylamino-phenyl)-5,5-dimethyl-1-pyridin-4-ylmethyl-imidazolidine-2,4-dione trifluoroacetate

Yield: 20mg
MS(ES⁺): m/e = 367
HPLC Retention time [min] = 0.71

### Example 8 : 3-(4-sec-Butyl-phenyl)-5,5-dimethyl-1-pyridin-4-ylmethyl-imidazolidine-2,4-dione trifluoroacetate

Yield: 46.4 mg
MS(ES⁺): m/e = 352
HPLC Retention time [min] = 1.61

### Example 9 : 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate

Yield: 16 mg
MS(ES⁺): m/e = 462
HPLC Retention time [min] = 1.38

### Example 10 : 5,5-Dimethyl-3-(4-piperidin-1-yl-phenyl)-1-pyridin-4-ylmethyl-imidazolidine-2,4-dione trifluoroacetate

Yield: 50 mg
MS(ES⁺): m/e = 379
HPLC Retention time [min] = 0.81

### Example 11 : 3-(4-Isopropyl-3-methyl-phenyl)-5,5-dimethyl-1-pyridin-4-ylmethyl-imidazolidine-2,4-dione trifluoroacetate

Yield: 46.1 mg
MS(ES⁺): m/e = 352
HPLC Retention time [min] = 1.44

### Example 12 : 3-(3-Fluoro-4-morpholin-4-yl-phenyl)-5,5-dimethyl-1-pyridin-4-ylmethyl-imidazolidine-2,4-dione trifluoroacetate

Yield: 15.3 mg
MS(ES⁺): m/e = 399
HPLC Retention time [min] = 1.06

### Example 13 : 3-[4-(Ethyl-isopropyl-amino)-phenyl]-5,5-dimethyl-1-pyridin-4-ylmethyl-imidazolidine-2,4-dione trifluoroacetate

Yield: 20.2 mg
MS(ES⁺): m/e = 381
HPLC Retention time [min] = 0.71

### Example 14 : 5,5-Dimethyl-1-pyridin-4-ylmethyl-3-[4-(thiophen-2-ylsulfanyl)-phenyl]-imidazolidine-2,4-dione trifluoroacetate

Yield: 28.9 mg
MS(ES⁺): m/e = 410
HPLC Retention time [min] = 1.52

### Example 15 : 3-(4-Benzenesulfonyl-3-chloro-phenyl)-5,5-dimethyl-1-pyridin-4-ylmethyl-imidazolidine-2,4-dione trifluoroacetate

Yield: 4.3 mg
MS(ES⁺): m/e = 471
HPLC Retention time [min] = 1.53

### Example 16 : 3-[4-(4-Fluoro-phenylsulfanyl)-phenyl]-5,5-dimethyl-1-pyridin-4-ylmethyl-imidazolidine-2,4-dione trifluoroacetate

Yield: 35.8 mg
MS(ES⁺): m/e = 422
HPLC Retention time [min] = 1.74

### Example 17 : 3-(4-Benzenesulfonyl-phenyl)-5,5-dimethyl-1-pyridin-4-ylmethyl-imidazolidine-2,4-dione trifluoroacetate

Yield: 39.8 mg
MS(ES⁺): m/e = 436
HPLC Retention time [min] = 1.28

If not stated otherwise LC/UV/MS experiments have been conducted with a Waters 1525 pump, a Waters 2488 UV detector, and a multiplexed ESI-TOF mass spectrometer (Micromass MUX-LCT) using YMC J*'*sphere H80 (30*2.1 mm, 4u, 80A) columns. UV data have been recorded at 220 nm and at 254 nm. For gradient separation, H2O+0.05% TFA and ACN+ 0.05% TFA are mixed in 95:5 (0 min) to 5:95 (3.4 min) to 5:95 (4.4 min) ratios at a flow rate of 1 ml min-1.

### EXAMPLE 18: PHARMACEUTICAL COMPOSITION

**Tablets were prepared corresponding to the following formula:**

| | |
|---|---|
| Product of Example 2 | 0.2 g |
| Excipient for a finished tablet weighing | 1 g |

| | |
|---|---|
| (details of the excipient: lactose, talc, starch, magnesium stearate). | |

### EXAMPLE 19: PHARMACEUTICAL COMPOSITION

**Tablets were prepared corresponding to the following formula:**

| | |
|---|---|
| Product of Example 7 | 0.2 g |
| Excipient for a finished tablet weighing | 1 g |

| | |
|---|---|
| (details of the excipient: lactose, talc, starch, magnesium stearate). | |

Pharmaceutical Composition Examples 18 and 19 above illustrate the present invention, it being understood that the same preparations can be made with other preferred products of formula (I) of the present invention, and form part of the present invention.

## Claims

1. Products of formula (I): 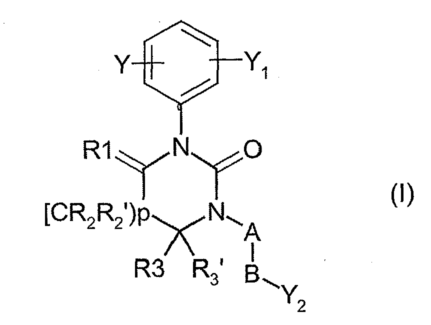
in which
R1 represents O or NH,
p represents an integer from 0 to 2,
Y and Y1, which may be identical or different, are such that one from among Y and Y1 is chosen from:
alkyl containing 1 to 7 carbon atoms optionally substituted with one or more radicals, which may be identical or different, chosen from halogen atoms, cycloalkyl and the hydroxyl radical;
3- to 7-membered cycloalkyl optionally substituted with one or more radicals, which may be identical or different, chosen from fluorine atoms and alkyl radicals containing 1 to 3 carbon atoms;
alkylamino, optionally substituted with one or more halogen atoms;
dialkylamino, optionally substituted with one or more radicals, which may be identical or different, chosen from halogen atoms and alkoxy radicals and in which the two alkyl residues may optionally form, together with the nitrogen atom to which they are attached, a 4- to 10-membered heterocycle optionally containing one or more other hetero atoms, which may be identical or different, chosen from O, N, NR4 and S and optionally substituted with one or more radicals, which may be identical or different, chosen from halogen atoms and alkyl and alkoxy radicals; phenylalkylamino optionally substituted on alkyl or phenyl; arylmercapto or heteroarylmercapto, optionally substituted with one or more radicals, which may be identical or different, chosen from halogen atoms and alkyl and alkoxy radicals;
and the other from among Y and Y1 is chosen from these same values and in addition from the following values: hydrogen; halogen; hydroxyl; nitro; CN; SF5; NR5R6; optionally substituted alkyl, alkenyl and alkoxy; optionally substituted aryl and heteroaryl; O-alkenyl ; O-alkynyl ; O-cycloalkyl ; S(O)n-alkyl ; S(O)n-alkenyl ; S(O) n-alkynyl ; S (O) n-cycloalkyl ; S (O) nNR5R6 ; free, salified or esterified carboxyl and CONR5R6 ;
p represents the integers 0, 1 and 2,
R2, R2' , R3 and R3', which may be identical or different, represent hydrogen, halogen, alkyl, alkenyl, alkynyl, cycloalkyl, cycloalkylalkyl, aryl and heteroaryl which are optionally substituted, or alternatively two of the residues R2, R2', R3 and R3' form, together with the carbon atom(s) to which they are attached, a carbocyclic or heterocyclic radical, these radicals being 3- to 10-membered and the heterocyclic radical containing one or more hetero atoms chosen from O, S, N and NR4, all these radicals optionally being substituted;
A represents a single bond; an alkylene radical; an alkenyl radical; alkynyl; CO; SO2; O; NH; NH-alkyl;
B represents a saturated or unsaturated monocyclic or bicyclic heterocyclic radical containing one or more hetero atoms, which may be identical or different, chosen from O, S, N and NR4, optionally substituted with one or more substituents, which may be identical or different, chosen from the values of Y2;
Y2 represents hydrogen; halogen; hydroxyl; cyano; alkyl; alkoxy; cycloalkyl; heterocycloalkyl; aryl; heteroaryl; -O-alkenyl; -O-alkynyl; -O-cycloalkyl; -S(O)n-alkyl; -S(O)n-alkenyl; -S(O)n-alkynyl; S(O)n-cycloalkyl; COOR13; -OCOR13; NR5R6; CONR5R6; S(O)n-NR5R6; -NR10-CO-R13; -NR10-SO2-R13; NH-SO2-NR5R6; -NR10-CO-NR5R6; -NR10-CS-NR5R6 and -NR10-COOR13; all these radicals being optionally substituted;
R4 represents a hydrogen atom or an alkyl, alkenyl, cycloalkyl, alkylCO, alkylSO₂ or aryle, all optionally substituted;
R5 and R6, which may be identical or different, are chosen from hydrogen; alkyl, alkenyl, alkynyl, cycloalkyl, cycloalkenyl, heterocycloalkyl, optionally substituted aryl and heteroaryl; or alternatively R5 and R6 form, with the nitrogen atom to which they are attached, a 3- to 10-membered heterocyclic radical containing one or more hetero atoms chosen from O, S, N and optionally substituted NR4;
all the above alkyl, alkenyl, alkynyl and alkoxy radicals being linear or branched and containing up to 6 carbon atoms ;
all the above cycloalkyl and heterocycloalkyl radicals containing up to 7 carbon atoms;
all the above aryl and heteroaryl radicals containing up to 10 carbon atoms;
all the above alkyl, alkenyl, alkynyl and alkoxy radicals cycloalkyl, heterocycloalkyl, aryl and heteroaryl radicals, carbocyclic and heterocyclic radicals, and also the ring formed by R5 and R6 with the atom to which they are attached being optionally substituted with one or more radicals, which may be identical or different, chosen from halogen atoms; cyano; hydroxyl; alkoxy; CF3; nitro; aryl; heteroaryl; -C(=O)-OR9; -C(=O)-R8; -NR11R12; -C(=O)-NR11R12; -N(R10)-C(=O)-R8 ; -N(R10)-C(=O)-OR9 N(R10)-C(=O)-NR11R12; -N(R10)-S(O)n-R8 -S (O) n-R8; -N (R10) -S (O) n-NR11R12 and -S (O) n-NR11R12 radicals;
all the above aryl and heteroaryl radicals also being optionally substituted with one or more radicals chosen from alkyl, phenylalkyl, alkoxy and alkylenedioxy radicals;
all the above cyclic radicals and also the ring formed by R5 and R6 with the atom to which they are attached being also optionally substituted with one or more radicals chosen from oxo and thioxo;
n represents an integer from 0 to 2,
R8 represents alkyl, alkenyl, cycloalkyl, cycloalkylalkyl, heterocycloalkyl, heterocycloalkyl-alkyl, aryl, arylalkyl, heteroaryl and heteroarylalkyl ; all these radicals being optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
R9 represents the values of R8 and hydrogen;
R10 represents hydrogen or alkyl;
R11 and R12, which may be identical or different, represent hydrogen; alkyl, cycloalkyl and phenyl optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
or alternatively R11 and R12 form, with the nitrogen atom to which they are attached, a 5- to 7-membered cyclic radical containing one or more hetero atoms chosen from O, S, N and NR14 and preferably a cyclic amine, optionally substituted with one or more radicals, which may be identical or different, chosen from halogen atoms and cyano, hydroxyl, alkoxy, alkyl, CF3, nitro, phenyl, phenylalkyl and free, salified, esterified or amidated carboxyl radicals;
R13, which may be identical to or different from R5 or R6, being chosen from the values of R5 or R6;
the said products of formula (I) being in all the possible racemic, enantiomeric and diastereoisomeric isomer forms, and also the addition salts with mineral and organic acids or with mineral and organic bases of the said products of formula (I).

2. Products of formula (I) according to Claim 1, corresponding to formula (Ia): 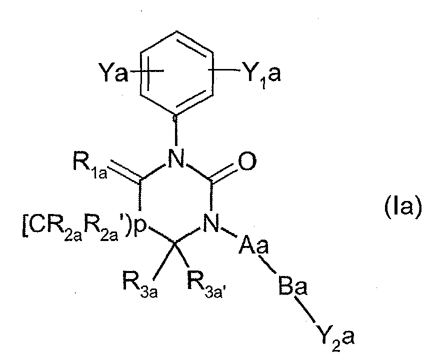
in which:
R1a stands for O or NH,
p represents an integer from 0 to 2,
Ya and Y1a, which may be identical or different, are such that one from among Ya and Y1a is chosen from:
alkyl containing 1 to 7 carbon atoms optionally substituted with one or more radicals, which may be identical or different, chosen from fluorine atoms and the radical hydroxyl; 3- to 7-membered cycloalkyl optionally substituted with one or more radicals, which may be identical or different, chosen from fluorine atoms, alkyl radicals containing 1 to 3 carbon atoms, cyclopropyl;
alkylamino, optionally substituted with one or more fluorine atoms; dialkylamino, optionally substituted with one or more radicals, which may be identical or different, chosen from fluorine atoms and alkoxy radicals and in which the two alkyl residues may optionally form, together with the nitrogen atom to which they are attached, a 4- to 10-membered heterocycle optionally containing one or more other hetero atoms, which may be identical or different, chosen from O, N, Nalkyl and S and optionally substituted with one or more radicals, which may be identical or different, chosen from fluorine atoms and alkyl and alkoxy radicals; phenylalkylamino optionally substituted on alkyl or phenyl; 5- to 6-membered phenylmercapto or heteroarylmercapto, optionally substituted with one or more radicals, which may be identical or different, chosen from halogen atoms and alkyl and alkoxy radicals;
and the other from among Ya and Y1a is chosen from these same values and in addition from the following values: hydrogen; halogen; hydroxyl ; nitro; CN; SF5 ; NR5aR6a; optionally substituted alkyl, alkenyl and alkoxy; O-allyl; O-propynyl; O-cycloalkyl; CF3; optionally substituted phenyl and heteroaryl; -S(O)n-alkyl, S(O)n-allyl; S(O)n-propynyl; SO2NR5aR6a; S(O)n-cycloalkyl; free, salified or esterified carboxyl; CONR5aR6a;
R2a, R2a', R3a, R3a' represent hydrogen, halogen, and alkyl, it being understood that two of the substituents R2a, R2a', R3a, R3a' can form, together with the carbon atom(s) to which they are attached, a 3- to 6-membered cycloalkyl or heterocycloalkyl radical containing a nitrogen atom, all these radicals being optionally substituted;
Aa represents a single bond; an alkylene radical; CO; SO2; O; NH; NH-alkyl;
Ba represents pyridyl, pyrimidinyl, quinolyl, azaindolyl, quinazolyl, thiazolyl, imidazolyl, pyrazolyl, furazanyl, isoxazolyl, morpholinyl, pyrrolidinyl, furyl, piperidyl, thienyl, chromenyl, oxochromenyl, indolyl, pyrrolyl, purinyl, benzoxazinyl, benzimidazolyl, indazolyl and benzofuryl radicals, these radicals being optionally substituted with one or more radicals chosen from the values of Y2a;
Y2a represents hydrogen; halogen; hydroxyl; alkyl; alkoxy ; cycloalkyl ; heterocycloalkyl ; aryl ; heteroaryl ; O-allyl ; O-propynyl ; O-cycloalkyl ; S(O)n-alkyl ; S(O)n-allyl; S(O)n-propynyl ; S(O)n-cycloalkyl ; COOR9a; OCOR8a ; -NR10a-CO-NR5aR6a ; NR5aR6a; CONR5aR6a ; S(O)n-R5aR6a ; NHCOR8a ; NH-S(O)nR8a ; NH-S(O)nCF3 ; NH-SO2-NR5aR6a, all these radicals being optionally substituted;
R4a represents a hydrogen atom or an alkyl, cycloalkyl, or phenyl radicals, all optionally substituted with one or more substituents, which may be identical or different, chosen from halogen atoms; hydroxyl; alkoxy; dialkylamino; aryl and heteroaryl radicals, these last two radicals optionally substituted with one or more substituents, which may be identical or different, chosen from halogen atoms and alkyl and alkoxy radicals,
R5a and R6a, which may be identical or different, are chosen from hydrogen, alkyl, alkenyl, cycloalkyl, cycloalkenyl, heterocycloalkyl, optionally substituted aryl and heteroaryl ; or alternatively R5a and R6a form, with the nitrogen atom to which they are attached, a 3-to 10-membered heterocyclic radical containing one or more hetero atoms chosen from O, S, N and optionally substituted NR4a;
all the above alkyl, alkenyl, alkynyl and alkoxy radicals being linear or branched and containing up to 6 carbon atoms;
all the above cycloalkyl and heterocycloalkyl radicals containing up to 7 carbon atoms;
all the above aryl and heteroaryl radicals containing up to 10 carbon atoms;
all the above alkyl, alkenyl, alkynyl, alkoxy, cycloalkyl, heterocycloalkyl, aryl, heteroaryl, carbocyclic and heterocyclic radicals being optionally substituted with one or more radicals, which may be identical or different, chosen from halogen atoms; cyano; hydroxyl; alkoxy; CF3; nitro; aryl; heteroaryl; -C(=O)-OR9a; -C(=O)-R8a; -NR11aR12a; -C(=O)-NR11aR12a; -N(R10a) - C(=O)-R8a; -N(R10a)-C(=O)-OR9a; N(R10a) -C(=O)-NR11aR12a; -N(R10a)-S(O)n-R8a; -S (O) n-R8a; -N(R10a)-S(O)n-NR11aR12a and -S(O)n-NR11aR12a radicals;
all the above aryl and heteroaryl radicals also being optionally substituted with one or more radicals chosen from alkyl, phenylalkyl and alkylenedioxy radicals;
n represents an integer from 0 to 2;
R8a represents alkyl, alkenyl, cycloalkyl, cycloalkylalkyl, heterocycloalkyl, heterocycloalkyl-alkyl, phenyl, phenylalkyl, heteroaryl and heteroarylalkyl ; all these radicals being optionally substituted with one or more radicals, which may be identical or different, chosen from halogen atoms and hydroxyl, alkoxy, alkyl, CF3, nitro, phenyl and free, salified, esterified or amidated carboxyl radicals;
R9a represents the values of R8 and hydrogen;
R10a represents hydrogen or alkyl;
R11a and R12a, which may be identical or different, represent hydrogen, alkyl, cycloalkyl, cycloalkylalkyl, phenyl and phenylalkyl optionally substituted with one or more radicals, which may be identical or different, chosen from halogen atoms and cyano, hydroxyl, alkoxy, alkyl, CF3, nitro, phenyl and free, salified, esterified or amidated carboxyl radicals;
or alternatively R11a and R12a form, with the nitrogen atom to which they are attached, a cyclic radical chosen from pyrrolidinyl, piperidyl, piperazinyl, morpholinyl, indolinyl, pyrindolinyl, tetrahydroquinolyl, thiazolidinyl and naphthyridyl; optionally substituted with one or more radicals, which may be identical or different, chosen from halogen atoms and alkyl, phenyl and phenylalkyl radicals;
the said products of formula (Ia) being in all the possible racemic, enantiomeric and diastereoisomeric isomer forms, and also the addition salts with mineral and organic acids or with mineral and organic bases of the said products of formula (Ia).

3. Products of formula (I) as defined in either of the preceding claims, such that p represents the integer 0, the other substituents of the said products of formula (I) having the values defined in any one of the preceding claims.

4. Products of formula (I) as defined in any one of the preceding claims, such that p represents the integer 1, the other substituents of the said products of formula (I) having the values defined in any one of the preceding claims.

5. Products of formula (I) as defined in any one of the preceding claims, such that p represents the integer 2, the other substituents of the said products of formula (I) having the values defined in any one of the preceding claims.

6. Products of formula (I) or (Ia) according to the preceding claims, corresponding to formula (Ib): 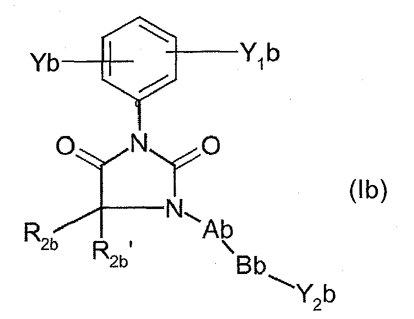
in which
Yb and Y1b, which may be identical or different, are such that:
Y is chosen from:
alkyl containing 1 to 6 carbon atoms optionally substituted with one or more fluorine atoms and OH;
3- to 6-membered cycloalkyl optionally substituted with one or more methyl radicals;
alkylamino;
dialkylamino, in which the two alkyl residues may optionally form, together with the nitrogen atom to which they are attached, a 5- or 6-membered heterocycle optionally containing one or more other hetero atoms, which may be identical or different, chosen from O, N, Nalkyl and S and optionally substituted with one or more radicals, which may be identical or different, chosen from fluorine atoms and alkyl radicals;
phenylalkylamino;
5- to 6-membered phenylmercapto or heteroarylmercapto, optionally substituted with one or more radicals, which may be identical or different, chosen from halogen atoms and alkyl radicals;
and the other from among Yb and Y1b is chosen from the same values and also from hydrogen; halogen; hydroxyl; oxo; nitro; NR5bR6b; optionally substituted alkyl, alkoxy -S-alkyl and phenyl; optionally substituted pyrazolyl, pyridyl, morpholino, pyrrolidinyl and piperazinyl ; free or esterified carboxyl;
R2b and R2b' represent hydrogen and alkyl, or two substituents R2b and R2b', can form, together with the carbon atom to which they are attached, a cycloalkyl radical containing from 3 to 6 carbon atoms, or form an azetidinyl, pyrrolidinyl or piperidyl radical.
Ab represents a single bond an alkylene radical;; O; NH; NH-alkyl;
Bb represents a heterocyclic radical chosen from 3- or 4-pyridyl; pyrimidinyl;; 3- or 4- quinolyl; azaindolyl; quinazolyl; indazolyl; thiazolyl; imidazolyl; pyrazolyl, furazanyl and isoxazolyl radicals ; these radicals being optionally substituted with one or more radicals chosen from the values of Yb;
Y2b represents hydrogen; halogen; hydroxyl ; alkyl ; alkoxy; cycloalkyl; heterocycloalkyl; phenyl; heteroaryl; O-cycloalkyl; S(O)n-alk; S(O)n-cycloalkyl; COOR9; OCOR8; -NR10b-CO-NR5bR6b; NR5R6; CONR5R6; S(O)n-R5R6; NHCOR8 and NH-S(O)nR8; all these radicals being optionally substituted;
R4b represents a hydrogen atom or an alkyl, cycloalkyl or phenyl radical;
R5b and R6b, which may be identical or different, are chosen from hydrogen, alkyl, alkenyl, cycloalkyl, heterocycloalkyl, optionally substituted phenyl and heteroaryl or alternatively R5b and R6b form, with the nitrogen atom to which they are attached, a 3- to 10-membered heterocyclic radical containing one or more hetero atoms chosen from O, S, N and optionally substituted NR4b;
all the above alkyl, alkenyl, alkynyl and alkoxy radicals being linear or branched and containing up to 6 carbon atoms;
all the above cycloalkyl and heterocycloalkyl radicals containing up to 7 carbon atoms;
all the above aryl and heteroaryl radicals containing up to 10 carbon atoms;
all the above optionally substituted radicals being optionally substituted with one or more radicals, which may be identical or different, chosen from halogen, cyano, hydroxyl, alkyl and alkoxy containing 1 to 4 carbon atoms, CF3, nitro, phenyl, carboxyl, free, salified, esterified with an alkyl radical or amidated with a radical NR11bR12b, -C(=O)-R9b, -NR11bR12b or -C(=O)-NR11bR12b;
R8b represents alkyl, cycloalkyl, cycloalkylalkyl and phenyl;
R9b, which may be identical to or different from R8b, represents hydrogen and the values of R8b;
R11b and R12b, which may be identical or different, represent hydrogen, alkyl, cycloalkyl and phenyl or alternatively R11b and R12b form, with the nitrogen atom to which they are attached, a pyrolidine, piperidinyl, morpholinyl or a piperazinyl radical optionally substituted with an alkyl, phenyl or phenylalkyl radical;
the said products of formula (Ib) being in all the possible racemic, enantiomeric and diastereoisomeric isomer forms, and also the addition salts with mineral and organic acids or with mineral and organic bases of the said products of formula (Ib).

7. Products of formula (I), (Ia) or (Ib) according to the preceding claims, corresponding to formula (Ic): 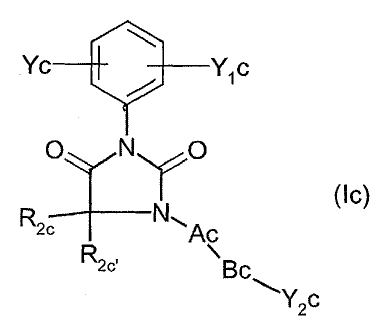
in which
Yc and Y1c, which may be identical or different, are such that one from among Yc and Y1c is chosen from alkyl especially such as methyl, ethyl, isopropyl, tert-butyl, sec-butyl, 1,2-dimethylpropyl, 1,1-dimethylpropyl optionally substituted with one or more fluorine atoms and OH; cyclopropyl or cyclobutyl especially such as 1-methylcyclopropyl, 2-methylcyclopropyl, 2,2-dimethylcyclopropyl, cyclobutyl, 2,2,3,3-tetrafluorocyclobutyl; di(C2-C4-alkyl)amino; phenylalkylamino; piperid-1-yl, thiomorpholin-4-yl, morpholin-4-yl, pyrrolidin-1-yl optionally substituted with one or more radicals chosen from fluorine atoms and alkyl radicals; phenylmercapto, optionally substituted with one or more halogen atoms; and the other from among Yc and Y1c is chosen from hydrogen; halogen; hydroxyl (oxo); nitro; NR5cR6c; optionally substituted alkyl, alkoxy, -S-alkyl and phenyl; optionally substituted pyrazolyl and pyridyl;
R2 and R2c'represent a hydrogen atom, alkyl of form together with the carbon atom bearing them a 3 to 6 membered cycloalkyl ring;
Ac represents a single bond, -O- or -CH2;
Bc represents a heterocyclic radical chosen from 3- or 4-pyridyl, pyrimidinyl, , 3- or 4- quinolyl, azaindolyl and quinazolyl, these radicals being optionally substituted with one or more radicals chosen from the values of Y2c,
Y2c represents hydrogen; halogen; alkyl; cycloalkyl; hydroxyl; alkoxy; NH2; NHalk; N(alk)2; NH-Phenyl-; NH-Heteroaryl; NH-CO-R5c; NH-CO-heteroaryl; NH-CO-NR5cR6c; and phenyl; all the alkyl, alkoxy, phenyl and heteroaryl radicals being optionally substituted;
R5c and R6c, which may be identical or different, represent hydrogen, alkyl, cycloalkyl and phenyl, which are optionally substituted, or alternatively R5c and R6c form, with the nitrogen atom to which they are attached, a cyclic radical chosen from pyrrolidinyl, piperidyl, piperazinyl, morpholinyl, piperazinyl, indolinyl, pyrindolinyl, tetrahydroquinoline and azetidine radicals, all these radicals being optionally substituted with one or more radicals chosen from alkyl, alkoxy and phenyl;
in all the above radicals, the alkyl, alkoxy and phenyl radicals are optionally substituted with one or more radicals chosen from halogen, OH, alk, Oalk, OCF3, S(O)n-CF3, CF3, NH2, NHAlk and N(alk)2; it beeing understood, that all dialkylamino radicals optionally can form a pyrrolidine, piperidine, morpholine or piperazine ring optionally substituted by one or more alkyl;
the said products of formula (Ic) being in all the possible racemic, enantiomeric and diastereoisomeric isomer forms, and also the addition salts with mineral and organic acids or with mineral and organic bases of the said products of formula (Ic).

8. Products of formula (I) as defined in any one of the preceding claims, corresponding to formula (Id): 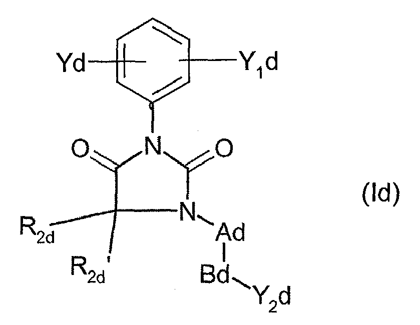
in which
Yd is chosen from the following values: alkyl especially such as methyl, ethyl, isopropyl, tert-butyl, sec-butyl, 1,2-dimethylpropyl, 1,1-dimethylpropyl optionally substituted with one or more fluorine atoms and OH; cyclopropyl or cyclobutyl such as, especially, 1-methylcyclopropyl, 2-methylcyclopropyl, 2,2-dimethylcyclopropyl, cyclobutyl, 2,2,3,3-tetrafluorocyclobutyl; di(C2-C4-alkyl)amino; phenylalkylamino; piperid-1-yl, thiomorpholin-4-yl, morpholin-4-yl or pyrrolidin-1-yl, optionally substituted with one or more radicals chosen from fluorine atoms and alkyl radicals ; phenylmercapto, optionally substituted with one or more halogen atoms; and Y1d is chosen from the following values: hydrogen; halogen; OH; oxo; nitro, NH2; free or esterified carboxyl; morpholino; alkyl, alkoxy, S-alkyl and phenyl optionally substituted;
in all these radicals, the alkyl and phenyl radicals are themselves optionally substituted with one or more radicals, which may be identical or different, chosen from halogen atoms and alkyl, alkoxy, OCF3, cyano, amino, alkylamino and dialkylamino radicals, and a phenyl radical, itself optionally substituted with one or more radicals, which may be identical or different, chosen from halogen atoms, and alkyl and alkoxy radicals;
R2d and R2d', which may be identical or different, are chosen from hydrogen or alkyl or form together with the atom bearing them a cyclopropyl or acyclobutyl ring;
Ad represents a single bond or CH2;
Bd represents a quinolyl or pyridyl radical optionally substituted with one or more radicals Y2d chosen from halogen, -OH, alk, -Oalk, -CO2H, -CO2alk, -NH2, NHalk, N(alk)2, -CF3, -OCF3 and phenyl; NH-phenyl; NH-heteroaryl NH-CO-phenyl, NH-CO-heteroaryl; NH-CO-NH-alkyl; NH-CO-N-dialkyl; NH-CO-NH-phenyl; the alkyl and phenyl radicals being themselves optionally substituted with one or more radicals chosen from halogen atoms and alkyl and alkoxy and dialkylamino radicals; it beeing understood, that all dialkylamino radicals optionally can form a 5- to 7 membered ring, optionally containing one more heteroatom or group from the series N,N-alkyl, O and S;
the said products of formula (Id) being in all the possible racemic, enantiomeric and diastereoisomeric isomer forms, and also the addition salts with mineral and organic acids or with mineral and organic bases of the said products of formula (Id).

9. Products of formula (I) as defined in any one of the preceding claims corresponding to formula (Id) in which:
Y is chosen from the following values: alkyl especially such as methyl, ethyl, isopropyl, tert-butyl, sec-butyl, 1,2-dimethylpropyl and 1,1-dimethylpropyl, optionally substituted with one or more fluorine atoms and OH; di(C2-C4-alkyl)amino; phenylalkylamino; piperid-1-yl, hiomorpholin-4-yl, morpholin-4-yl, pyrrolidin-1-yl; and Y1 is chosen from the following values: hydrogen, halogen, nitro, NH2 and alkyl;
R2 and R2' , which may be identical or different, are chosen from hydrogen or alkyl;
A represents CH2;
B represents a quinolyl or pyridyl radical;
the said products of formula (I) being in all the possible racemic, enantiomeric and diastereoisomeric isomer forms, and also the addition salts with mineral and organic acids or with mineral and organic bases of the said products of formula (I).

10. Products of formula (I) as defined in any one of Claims 1 to 9, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3-[4-(4-Fluoro-phenylsulfanyl)-phenyl]-5,5-dimethyl-1-pyridin-4-ylmethyl-imidazolidine-2,4-dione trifluoroacetate
- 3-(4-Isopropyl-3-nitro-phenyl)-5,5-dimethyl-1-pyridin-4-ylmethyl-imidazolidine-2,4-dione
- 3-(3-Amino-4-isopropyl-phenyl)-5,5-dimethyl-1-pyridin-4-ylmethyl-imidazolidine-2,4-dione
the said products of formula (I) being in all the possible racemic, enantiomeric and diastereoisomeric isomer forms, and also the addition salts with mineral and organic acids or with mineral and organic bases of the said products of formula (I).

11. Products of formula (I) as defined in any one of Claims 1 to 9, the names of which are given hereinbelow:
- 3-(4-tert-Butyl-phenyl)-5,5-dimethyl-1-quinolin-4-ylmethyl-imidazolidine-2,4- dione
- 3-(4-Isopropyl-phenyl)-5,5-dimethyl-1-quinolin-4-ylmethyl-imidazolidine-2,4- dione
- 3-(4-Diethylamino-phenyl)-5,5-dimethyl-1-pyridin-4-ylmethyl-imidazolidine-2,4-dione trifluoroacetate
- 3-(4-sec-Butyl-phenyl)-5,5-dimethyl-1-pyridin-4-ylmethyl-imidazolidine-2,4-dione trifluoroacetate
- 5,5-Dimethyl-1-pyridin-4-ylmethyl-3-[4-(2,2,2-trifluoro-1-hydroxy-1-trifluoromethyl-ethyl)-phenyl]-imidazolidine-2,4-dione trifluoroacetate
- 5,5-Dimethyl-3-(4-piperidin-1-yl-phenyl)-1-pyridin-4-ylmethyl-imidazolidine-2,4-dione trifluoroacetate
- 3-(4-Isopropyl-3-methyl-phenyl)-5,5-dimethyl-1-pyridin-4-ylmethyl-imidazolidine-2,4-dione trifluoroacetate
- 3-[4-(Ethyl-isopropyl-amino)-phenyl]-5,5-dimethyl-1-pyridin-4-ylmethyl-imidazolidine-2,4-dione trifluoroacetate
- 5,5-Dimethyl-1-pyridin-4-ylmethyl-3-[4-(thiophen-2-ylsulfanyl)-phenyl]-imidazolidine-2,4-dione trifluoroacetate
- 3-[4-(4-Fluoro-phenylsulfanyl)-phenyl]-5,5-dimethyl-1-pyridin-4-ylmethyl-imidazolidine-2,4-dione trifluoroacetate
the said products of formula (I) being in all the possible racemic, enantiomeric and diastereoisomeric isomer forms, and also the addition salts with mineral and organic acids or with mineral and organic bases of the said products of formula (I).

12. As medicinal products, the products of formula (I) as defined in any one of Claims 1 to 11, and also the prodrugs thereof, the said products of formula (I) being in all the possible racemic, enantiomeric and diastereoisomeric isomer forms, and also the pharmaceutically acceptable addition salts with mineral and organic acids or with mineral and organic bases of the said products of formula (I).

13. As medicinal products, the products of formula (Ia), (Ib), (Ic) or (Id) as defined in the preceding claims, and also the prodrugs thereof, the said products of formula (Ia), (Ib), (Ic) or (Id) being in all the possible racemic, enantiomeric and diastereoisomeric isomer forms, and also the pharmaceutically acceptable addition salts with mineral and organic acids or with mineral and organic bases of the said products of formula (Ia), (Ib), (Ic) or (Id).

14. As medicinal products, the products as defined in Claim 10 or 11 and also the prodrugs thereof, and also the pharmaceutically acceptable addition salts of these products with mineral and organic acids or with mineral and organic bases.

15. Pharmaceutical compositions containing, as active principle, at least one of the medicinal products as defined in Claims 12 to 14.

16. Pharmaceutical compositions according to Claim 15 **characterized in that** they are used as medicinal products, in particular for cancer chemotherapy.

17. Pharmaceutical compositions according to Claim 16, also containing active principles of other chemotherapy medicinal products for combating cancer.

18. Use of products of formula (I) as defined in any one of the claims, or of pharmaceutically acceptable salts of the products of formula (I) for the preparation of medicinal products for inhibiting the activity of protein kinases and especially of a protein kinase.

19. Use of products of formula (I) as defined in the preceding claim or of pharmaceutically acceptable salts of the products of formula (I), in which the protein kinase is a protein tyrosine kinase.

20. Use of products of formula (I) as defined in any one of the preceding claims, or of pharmaceutically acceptable salts of the products of formula (I), in which the protein kinase is chosen from the following group: IGF1, Raf, EGF, PDGF, VEGF, Tie2, KDR, Flt1-3, FAK, Src, Abl, cKit, cdk1-9, Auroral-2, cdc7, Akt, Pdk, S6K, Jnk, IR, FLK-1, FGFR1, FGFR2, FGFR3, FGFR4, FGFR5 , PLK, Pyk2, CDK7, CDK2 et EGFR.

21. Use of products of formula (I) as defined in any one of the claims, or of pharmaceutically acceptable salts of the products of formula (I), in which the protein kinase is chosen in the following group: IGF1, cdc7, Auroral-2, Src, Jnk, FAK, KDR, IR, Tie2, CDK7, CDK2 et EGFR.

22. Use of products of formula (I) as defined in any one of the claims, or of pharmaceutically acceptable salts of the products of formula (I), in which the protein kinase is IGF1R.

23. Use of products of formula (I) as defined in any one of the claims, or of pharmaceutically acceptable salts of the said products of formula (I) in which the protein kinase is FAK or AKT.

24. Use of products of formula (I) as defined in any one of the claims, or of pharmaceutically acceptable salts of the products of formula (I), in which the protein kinase is in a cell culture.

25. Use of products of formula (I) as defined in any one of the claims, or of pharmaceutically acceptable salts of the products of formula (I), in which the protein kinase is in a mammal.

26. Use of products of formula (I) as defined in any one of the claims or of pharmaceutically acceptable salts of the products of formula (I), for the preparation of a medicinal product for preventing or treating a disease **characterized by** deregulation of the activity of a protein kinase.

27. Use of products of formula (I) according to the preceding claim, in which the disease to be prevented or treated is in a mammal.

28. Use of products of formula (I) as defined in any one of the claims or of pharmaceutically acceptable salts of the products of formula (I), for the preparation of a medicinal product for preventing or treating a disease belonging to the following group: disorders of blood vessel proliferation, fibrotic disorders, disorders of mesangial cell proliferation, acromegaly, metabolic disorders, allergies, asthma, Crohn's disease, thrombosis, diseases of the nervous system, retinopathy, psoriasis, rheumatoid arthritis, diabetes, muscle degeneration, aging, age related macula degeneration, oncology diseases and cancer.

29. Use of products of formula (I) as defined in any one of the claims or of pharmaceutically acceptable salts of said products of formula (I) for the preparation of a medicinal product for treating oncology diseases.

30. Use of products of formula (I) as defined in any one of the claims or of pharmaceutically acceptable salts of said products of formula (I) for the preparation of a medicinal product for treating cancer.

31. Use of products of formula (I) as claimed in the preceding claim, in which the disease to be treated is a cancer of solid tumours.

32. Use of products of formula (I) as claimed in the preceding claim, in which the disease to be treated is a cancer that is resistant to cytotoxic agents.

33. Use of products of formula (I) as defined in any one of the claims, or of pharmaceutically acceptable salts of said products of formula (I), for the preparation of a medicinal product for treating cancers, among which are breast cancer, stomach cancer, cancer of the colon, lung cancer, cancer of the ovaries, cancer of the uterus, brain cancer, cancer of the kidney, cancer of the larynx, cancer of the lymphatic system, cancer of the thyroid, cancer of the urogenital tract, cancer of the tract including the seminal vesicle and prostate, bone cancer, cancer of the pancreas and melanomas.

34. Use of products of formula (I) as claimed in the preceding claim, in which the disease to be treated is a breast cancer, cancer of the colon or lung cancer.

35. Use of products of formula (I) as defined in any one of the claims or of pharmaceutically acceptable salts of said products of formula (I) for the preparation of a medicinal product for cancer chemotherapy.

36. Use of products of formula (I) as defined in any one of the claims or of pharmaceutically acceptable salts of said products of formula (I) for the preparation of medicinal products for cancer chemotherapy, used alone or in combination.

37. Use of products of formula (I) as defined in any one of the claims or of pharmaceutically acceptable salts of said products of formula (I) for the preparation of medicinal products for use alone or in combination with chemotherapy or radiotherapy or alternatively in combination with other therapeutic agents.

38. Use of products of formula (I) as claimed in the preceding claim, in which the therapeutic agents may be commonly used antitumour agents.

39. Products of formula (I) as defined in any one of the claims, as protein kinase inhibitors, said products of formula (I) being in any possible racemic, enantiomeric or diastereoisomeric isomer form, and also the addition salts of said products of formula (I) with pharmaceutically acceptable mineral and organic acids or with pharmaceutically acceptable mineral and organic bases, and also the prodrugs thereof.

40. Products of formula (I) as defined in any one of the claims, as IGF1R inhibitors.

41. Products of formula (Id) as defined in the claims, as IGF1R inhibitors.
